(19) Europäisches Patentamt
European Patent Office
Office européen des brevets

(11) **EP 2 274 080 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(21) Anmeldenummer: **09731848.9**

(22) Anmeldetag: **10.02.2009**

(51) Int Cl.:
*B01D 69/02* (2006.01)    *B01D 71/10* (2006.01)
*B01D 67/00* (2006.01)    *B01J 20/28* (2006.01)
*B01J 20/26* (2006.01)    *B01D 15/32* (2006.01)
*B01J 20/285* (2006.01)    *C07K 1/30* (2006.01)
*C08B 15/10* (2006.01)

(86) Internationale Anmeldenummer:
**PCT/EP2009/000914**

(87) Internationale Veröffentlichungsnummer:
**WO 2009/127286 (22.10.2009 Gazette 2009/43)**

(54) **HYDROPHOBE CELLULOSE-MEMBRAN, VERFAHREN ZU IHRER HERSTELLUNG UND IHRE VERWENDUNG IN DER HYDROPHOBEN INTERAKTIONSCHROMATOGRAPHY**

HYDROPHOBIC CELLULOSE MEMBRANE, METHOD FOR THE PRODUCTION THEREOF, AND USE OF SAME IN HYDROPHOBIC INTERACTION CHROMATOGRAPHY

MEMBRANE DE CELLULOSE HYDROPHOBE, PROCÉDÉ POUR SA PRODUCTION ET SON UTILISATION DANS LA CHROMATOGRAPHIE D'INTERACTION HYDROPHOBE

(84) Benannte Vertragsstaaten:
**AT BE BG CH CY CZ DK EE ES FI FR GB GR HR HU IE IS IT LI LT LU LV MC MK MT NL NO PL PT RO SE SI SK TR**

(30) Priorität: **14.04.2008 DE 102008018734**

(43) Veröffentlichungstag der Anmeldung:
**19.01.2011 Patentblatt 2011/03**

(73) Patentinhaber: **Sartorius Stedim Biotech GmbH 37079 Göttingen (DE)**

(72) Erfinder: **FABER, René 37077 Göttingen (DE)**

(74) Vertreter: **Müller-Boré & Partner Patentanwälte PartG mbB Friedenheimer Brücke 21 80639 München (DE)**

(56) Entgegenhaltungen:
**WO-A-03/015902        WO-A-2007/017085**
**WO-A-2008/095709        DE-A1- 4 323 913**
**US-A- 6 103 121**

EP 2 274 080 B1

**Beschreibung**

Die vorliegende Erfindung betrifft Adsorbentien für die adsorptive Stofftrennung in flüssigen Medien aufgrund hydrophober Wechselwirkung sowie Vorrichtungen, welche die erfindungsgemäßen Adsorbentien umfassen. Insbesondere betrifft die vorliegende Erfindung eine Cellulosehydrat-Membran, Verfahren zu ihrer Herstellung sowie ihre Verwendung in der hydrophoben Interaktionschromatographie.

Der Erfindungsbeschreibung liegen folgende Begriffsbestimmungen und Sachverhalte zugrunde, wobei unter "Durchfluss" die hydraulische Permeabilität verstanden wird.

Als Adsorptionsmembranen werden flächige Adsorbentien mit von der einen Seite zur anderen Seite durchgehenden Poren bezeichnet. Adsorbentien sind poröse Feststoffe, die über als Liganden bezeichnete funktionelle Oberflächengruppen mit bestimmten Komponenten von Fluiden selektiv Bindungen eingehen können. Zielsubstanz(en) und/oder Kontaminant(en) werden erfindungsgemäß als Adsorbenden bezeichnet, wobei es sich auch um mehrere unterschiedliche Substanzen handeln kann. Adsorbenden können Einzelmoleküle, Assoziate oder Partikel sein, bei denen es sich vorzugsweise um Proteine oder andere Substanzen biologischen Ursprungs handelt.

Beispielhaft können als mit den bzw. dem Adsorbenden wechselwirkende Liganden Ionenaustauscher, Chelatbildner und Schwermetallchelate, thiophile, hydrophobe Liganden verschiedener Kettenlängen und Konfigurationen, Reversed Phase-Systeme, Farbstoffliganden, Affinitätsliganden, Aminosäuren, Coenzyme, Cofaktoren und deren Analoga, Substrate und deren Analoga, endokrine und exokrine Substanzen, wie Hormone und hormonähnlich wirkende Wirkstoffe, Effektoren und deren Analoga, Enzym-Substrate, Enzym-Inhibitoren und deren Analoga, Fettsäuren, Fettsäurederivate, konjugierte Fettsäuren und deren Analoga, Nukleinsäuren, wie DNA, RNA und deren Analoga und Derivate (einzel-, doppel-und/oder mehrsträngig), sowie Peptidnukleinsäuren und deren Derivate, Viren, Virenpartikel, Monomere und deren Analoga und Derivate, Oligo-bis-Polymere und deren Analoga und Derivate, hochmolekulare Kohlenhydrate, die linear oder verzweigt, nicht-substituiert oder substituiert sein können, polymere Glycokonjugate, wie Heparin, Amylose, Cellulose, Chitin, Chitosan und deren Mono- und Oligomere und Derivate und Analoga davon, Lignin und dessen Derivate und Analoga, andere biologisch-chemische Liganden, wie Oligo- und Polypeptide, z.B. Proteine und ihre Oligomere, Multimere, Untereinheiten sowie Teile davon, insbesondere Lectine, Antikörper, Fusionsproteine, Haptene, Enzyme und Untereinheiten sowie Teile davon, Strukturproteine, Rezeptoren und Effektoren sowie Teile davon, des weiteren Xenobiotika, Pharmazeutika und pharmazeutische Wirkstoffe, Alkaloide, Antibiotika, Biomimetika usw. genannt werden.

Ein Adsorbens kann gleichzeitig auch zwei oder mehrere Arten der funktionellen Gruppen auf seiner inneren und äußeren Oberfläche tragen.

Die Bindung der Adsorbenden an das Adsorbens kann reversibel oder irreversibel sein, in jedem Fall ermöglicht sie ihre Abtrennung von den Fluiden, bei denen es sich im allgemeinen um wässrige Flüssigkeiten handelt und die im folgenden Medien genannt werden. Unter dem Begriff "Elution" werden die Desorption und die damit einhergehenden Spülschritte etc. zusammengefasst, und die zur Elution verwendete Flüssigkeit ist das "Eluens". Die Komponenten können eine oder mehrere Zielsubstanzen und/oder einen oder mehrere Kontaminanten darstellen. "Zielsubstanzen" sind Wertstoffe, die aus dem Medium in angereicherter oder reiner Form gewonnen werden sollen. "Kontaminanten" sind Stoffe, deren Abwesenheit aus technischen, regulatorischen oder sonstigen Gründen erforderlich oder wünschenswert ist. Für die Entfernung von Kontaminanten, die als "negative Adsorption" bezeichnet wird, kann (darf) die Adsorption irreversibel verlaufen, wenn das Adsorbens nur einmal verwendet werden soll. Bei der Adsorption der Zielsubstanz(en) muß der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Diesen Vorgang bezeichnet man als adsorptive Stofftrennung oder Chromatographie. Herkömmliche Adsorbentien für die Chromatographie sind partikulär geformt und werden in Form von Schüttungen in Säulen betrieben. Im Gegensatz dazu erfolgt die Anwendung von Adsorptionsmembranen im allgemeinen in Modulen, deren Bauformen den meist in der Membranfiltration üblichen entsprechen (z.B. Wickelmodul, Stapelmodul etc.). Die Anforderungen an die mechanische Festigkeit gleichen den an Filtrationsmembranen zu stellenden und sind damit wesentlich höher, als bei partikulären Adsorbentien, für die sich fragile Trägermaterialien, wie Gele von Dextran oder Agarose so universell etabliert haben, daß sich für sie der Begriff "Gele" als Synonym eingebürgert hat. Allen Membranen und Gelen ist dagegen eine Grundanforderung gemeinsam, und zwar die nach möglichst niedriger unspezifischer Adsorption.

Die Ausführung chromatographischer Trennung mit Hilfe von Adsorptionsmembranen wird auch Membranchromatographie genannt und sämtliche der in der Chromatographie bekannten synthetischen und natürlichen Liganden können in gleicher Weise auch für Adsorptionsmembranen eingesetzt werden. Der Bindung des Liganden an den Träger kann eine "Aktivierung" des Trägers vorausgehen, also die Einführung von reaktiven, funktionellen Gruppen, die zur spontanen Bindung des Liganden befähigt sind. Seltener weist der Ligand selbst eine reaktive Gruppe auf, wie z.B. die als Farbstoffliganden dienenden Reaktivfarbstoffe aus der Textilindustrie. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Die Menge an Adsorbend, bezogen auf die Menge des Adsorbens, die pro Beladung im Gleichgewicht mit dem Medium gebunden wird, d.h. die spezifische Bindungskapazität des Adsorbens, ist bei gegebener Ligandendichte proportional zu seiner spezifischen Oberfläche. Die spezifische Oberfläche von porösen Strukturen steigt mit abnehmender Porengröße, infolgedessen steigt auch ihre spezifische Bindungskapazität, solange die Ausschlußgrenze der Poren, also diejenige Molmasse, oberhalb derer ein Eintritt eines Moleküls möglich ist, die Molmasse des Adsorbenden nicht unterschreitet.

Trennmaterialien für die Umkehrphasenchromatographie (Reversed Phase Chromatography oder RP-Chromatographie) und die hydrophobe Interaktionschromatographie (HIC) und ihre Herstellung sind bekannt, z.B. aus DE 43 23 913 A1. Es handelt sich um Trennmaterialien für die hydrophobe Chromatographie auf der Grundlage von hydroxylgruppenhaltigen Basisträgern, auf deren Oberflächen Polymere kovalent gebunden sind.

Als Träger sind im Stand der Technik unvernetzte oder vernetzte Polysaccharide, wie z.B. Sepharose-Gele der Firma GE Healthcare sowie vernetzte Poly(meth)acrylate in Form von porösen kugelförmigen Gelen gebräuchlich. Diese sind meistens mit Alkyl- oder Arylresten, beispielsweise Butyl-, Hexyl- oder Phenylresten substituiert. Ein übliches Verfahren zur Herstellung dieser Adsorbentien beruht auf der Umsetzung eines Trägers, der mit Oxirangruppen aktiviert ist, mit einem Alkohol in Gegenwart von Bortrifluoridetherat. Andere Herstellverfahren beruhen auf der Umsetzung eines hydroxylgruppenhaltigen Trägers mit einem oxirangruppenhaltigen Molekül in Gegenwart von Bortrifluoridetherat. Andere Verfahren zur Herstellung hydrophober Adsorbentien basieren auf der Umsetzung von oxiranhaltigen aktivierten Trägern mit Aminen.

Für die adsorptive Stofftrennung aufgrund hydrophober Wechselwirkung sind zwei Gruppen von Liganden, welche auf Trägern aufgebracht sind, gebräuchlich:

Liganden für die Umkehrphasenchromatographie, welche zu hoher Hydrophobizität der Matrix führen, werden hauptsächlich zur Trennung kleiner Adsorbenden verwendet. Gebräuchliche Liganden bestehen aus $C_8$- oder $C_{18}$-Alkylresten.

Liganden für die hydrophobe Interaktionschromatographie (HIC), welche zu geringerer Hydrophobizität der Matrix führen, werden hauptsächlich zur Trennung großer Adsorbenden, wie Proteine in wässrigen Medien, verwendet. Oft verwendete Liganden sind z.B. $C_4$-Alkylreste, $C_6$-Alkylreste, Phenylreste oder Polymere, wie Polyethylenglycol oder Polypropylenglycol.

Werden partikuläre, d.h. teilchenförmige Adsorbentien zur Herstellung eines Adsorbens verwendet, ergibt sich eine Reihe von Nachteilen:

1. Die Beschickung eines Adsorbers mit dem entsprechenden Adsorbens ist aufwendig, da jede Ungleichmäßigkeit, Kanalbildung und dergleichen vermieden werden muß. Hinzu kommen unvermeidliche, nachteilige Randeffekte zwischen Adsorbens und Adsorbergehäuse.

2. Es besteht ein Antagonismus zwischen Druckabfall und Transportkinetik in der Weise, daß letztere durch abnehmende Partikelgrößen begünstigt, gleichzeitig aber der Druckabfall erhöht wird.

3. Weitere Nachteile von flüssigkeitschromatographischen Stofftrennungen mit "partikulären" Adsorbentien bestehen in ihrer langsamen Betriebsweise auf Grund der Diffusionslimitierung des Stofftransports.

Die langsame Betriebsweise von konventionellen Chromatographiesäulen kann zu langen Verweilzeiten von Zielmolekülen in der Säule und daraus resultierend zur irreversiblen Schädigung der Zielmoleküle (z. B. durch Änderung der dreidimensionalen Proteinstruktur) führen.

Für die hydrophobe Interaktionschromatographie können auch Membranen eingesetzt werden. Die in dem Stand der Technik hierfür bekannten Membranen basieren meistens auf gepfropften Hohlfasermembranen. Siehe hierzu z.B. die Artikel von Kubota in Reactive & Functional Polymers 29 (1996) 115-122, "Control of phenyl-group site introduced on the graft chain for hydrophobic interaction chromatography" und von Kubota in Journal of Chromatography A, 718 (1995) 27-34, "Preparation of a hydrophobic porous membrane containing phenyl groups and its protein adsorption performance".

Eine mittels Elektronenstrahlen mit Glycidylmethacrylat (GMA) gepfropfte 0,2 $\mu$m Polyethylen-(PE)-Hohlfasermembran (Innendurchmesser 0,7 mm, Außendurchmesser 1,2 mm, Wandstärke 250 $\mu$m) wurde zum Teil mit Phenol umgesetzt und restliche Epoxidgruppen wurden mit Schwefelsäure hydrolysiert. Die so hergestellte phenylmodifizierte Membran wurde auf die Bindung von Rinderserumalbumin (RSA) untersucht. Die Phenylligandendichte betrug 0,53-0,64 mol/kg bei 14-17 % Phenolteilumsetzung. Zur Adsorption wurde eine Lösung von 0,2 mg/ml RSA in 0,07 M Phosphatpuffer pH 7,4, 2 M $(NH_4)_2SO_4$ verwendet. Gewaschen wurde mit einem 0,07 M Phosphatpuffer pH 7,4, 2 M $(NH_4)_2SO_4$ und die Elution erfolgte mit einem 0,07 M Phosphatpuffer pH 7,4.

Die Bindungskapazität betrug 33-37 mg RSA/g Adsorbens bei einer Wiederfindung der Zielsubstanz im Eluat von bis zu 80 %. Die Basismembran (gepfropft und vollständig hydrolisiert) zeigte etwa 50 % der Bindungskapazität der mit Phenol umgesetzten Membran (Umsetzungsgrad 15 %).

In dem Artikel von Kawai in Journal of Chromatography B, 790 (2003) 131-142, "Protein binding to polymer brush, based on ion-exchange, hydrophobic and affinity interactions" ist eine mittels Elektronenstrahlen mit Glycidylmethacrylat (GMA) gepfropfte Polyethylen-(PE)- oder Polypropylen-(PP)-Hohlfasermembran beschrieben, die mit Phenol und Butanol umgesetzt wurde. Die Bindungskapazität für RSA entspricht der theoretisch berechneten "monolayer"-Bindung. Es trat keine Quellung der gepfropften Schicht auf.

In dem Artikel von Saito in Journal of Chromatography, 586 (1991) 27-33, "Protein adsorption capacity of a porous phenylalanine-containing membrane based on a polyethylene matrix" ist eine mittels Elektronenstrahlen mit Glycidylmethacrylat (GMA) gepfropfte PE-Hohlfasermembran (Innendurchmesser 0,62 mm, Außendurchmesser 1,24 mm, Wandstärke 310 $\mu$m) beschrieben, die über Epoxidgruppen gebundene Phenylalaningruppen aufweist.

Zur Adsorption wurde Rinder-g-Globulin in 0,01 M TRIS/HCl pH 8,0, 3,3 M NaCl verwendet und die Elution erfolgte mit 0,01 M TRIS/HCl pH8,0, 1-2 M NaCl.

Die Bindungskapazität betrug 30 bis 50 mg Rinder-g-Globulin/g Adsorbens, bei einer Wiederfindung der Zielsubstanz im Eluat von bis zu 80 %.

Weiter sind im Stand der Technik flächig ausgebildete Membranen bekannt, die keine funktionellen Gruppen tragen, aber aufgrund ihrer hydrophoben Eigenschaften für die HIC in Kombination mit Filtration verwendet werden können.

In dem Artikel von Ghosh in Journal of Immunological Methods 314 (2006) 1-8, "Purification of humanized monoclonal antibodies by membrane-based hybrid bioseparation technique" ist eine nicht modifizierte Polyvinylidenfluorid-(PVDF)-Membran der Firma Millipore mit einem Porendurchmesser von 0,22 $\mu$m für eine Kombination von Filtration und hydrophober Interaktionschromatographie zur Antikörperreinigung beschrieben.

Im Stand der Technik sind weiterhin flächig ausgebildete Membranen der Firma PolyAn bekannt, die hydrophobe funktionelle Gruppen tragen und als HIC-Membranen bezeichnet werden. Die Auswirkung des hydrophoben Liganden auf die Bindung ist jedoch gering und die Bindungskapazität ist im Vergleich zu der Basismembran (d.h. Membran ohne Ligand) nur bis zu 1,8 mal höher.

Bis jetzt haben sich Membranen für hydrophobe Interaktionschromatographische, d.h. HIC-Anwendungen, nicht im industriellen Maßstab durchgesetzt. Die im Stand der Technik bekannten, makroporösen, flächigen Membranen, die mit hydrophoben Liganden modifiziert sind, zeigen keine ausreichenden Bindungskapazitäten.

Die Beschichtung von makroporösen Trägerstrukturen mit einer ligandentragenden Polymerschicht hat sich für Ionenaustauschermembranen durchgesetzt. Die ionischen Gruppen in den Poren der Ionenaustauscher-Membranen stoßen sich bei niedrigen Ionenstärken gegenseitig ab, so daß sich eine dreidimensionale Hydrogel-Struktur in den Poren des Trägers bilden kann. Dieser Effekt, auch als Polyelektrolyteffekt bekannt, tritt im Falle von hydrophoben Liganden nicht auf. Die hydrophobe Schicht kollabiert bei hohen Ionenstärken, was die Bildung der dreidimensionalen Struktur verhindert (siehe E. Müller, Chem. Eng. Technol. 2005, 28, No. 11). So ist es bisher nicht möglich, hohe Bindungskapazitäten, wie sie bei Ionenaustauscher-Membranen bekannt sind, auch mit hydrophoben Liganden oder Affinitätsliganden zu erreichen.

WO 03/015902 A2 betrifft eine Cellulosemembran, wobei die Membran aus einem Material gegossen worden ist, das ein Cellulosepolymer und ein Lösungsmittel umfasst, wobei die Membran eine erste poröse Fläche mit einem ersten durchschnittlichen Porendurchmesser, eine zweite poröse Fläche mit einem zweiten durchschnittlichen Porendurchmesser, und eine poröse Trägerstruktur dazwischen aufweist, wobei die Trägerstruktur ein netzförmiges Netzwerk aus Strömungskanälen umfasst, wobei der erste und der zweite durchschnittliche Porendurchmesser eine Asymmetrie von mindestens etwa 2:1 aufweisen, und wobei die porösen Flächen und die poröse Trägerstruktur ein Netzwerk von strukturellen Oberflächen aufweisen, die einen Filterstrom kontaktieren können.

US 6,103,121 betrifft ein Verfahren zur Herstellung eines Sorptionsmittels auf Membranbasis, das für eine Metallionenkomplexierung geeignet ist, umfassend das selektive Hydrolysieren einer mikroporösen Membran, die aus einem polyacetylierten Cellulosematerial aufgebaut ist, um eine Oberflächenschicht der Membran zu deacetylieren und freie Hydroxylgruppen freizulegen, das Oxidieren der freigelegten Hydroxylgruppen zu Aldehydgruppen und das Binden einer Polyaminosäure an die Cellulosemembran mittels der Aldehydgruppen.

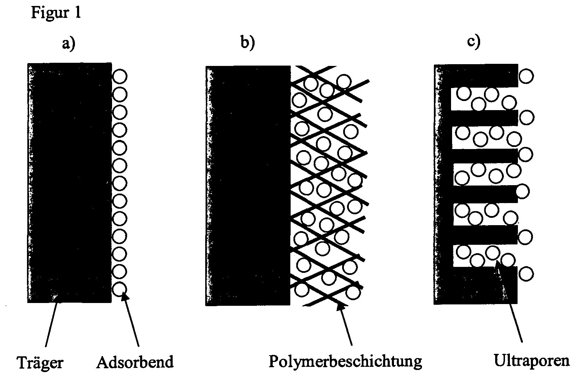

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Da sich die Struktur der Membran im simultan ablaufenden Verseifungs- und Vernetzungsprozess nicht ändert, ist davon auszugehen, daß die Adsorption von Adsorbenden an der Oberfläche der Mikroporen des Trägers, die der Oberfläche der Augangs-Celluloseester-Membran entspricht. stattfindet (vgl. fig. 1a).

WO 2008/095709 A1, welche Stand der Technik gemäß Art. 54(3) EPÜ darstellt, beschreibt ein Verfahren zur Herstellung einer porösen, vernetzten, geladenen Cellulosepolymermembran, wobei die Cellulosepolymermembran in Gegenwart einer Base vernetzt wird und anschließend geladene Liganden in diese Membran eingeführt werden, und die so erhaltene Membran in Wasser eine Quellung von 40 bis 250 Vol.-% aufweist.

Der vorliegenden Erfindung liegt daher die Aufgabe zugrunde, flächig ausgebildete Adsorbentien, d.h. Membranen mit verbesserten Eigenschaften, insbesondere mit höherer Bindungskapazität für die hydrophobe Interaktionschromatogrphie bereitzustellen sowie ein kostengünstiges und umweltfreundliches Verfahren zur Herstellung solcher Adsorbentien anzugeben.

Diese Aufgaben werden durch die in den Ansprüchen gekennzeichneten Gegenstände gelöst.

Gegenstand der vorliegenden Erfindung sind somit Trennmaterialien bzw. Adsorbentien für die hydrophobe Interaktionschromatographie auf der Grundlage von flächig bzw. flachförmig ausgebildeten, porösen Basisträgern (Membranen), auf deren Oberfläche funktionelle Gruppen immobilisiert sind, d.h. hydrophobe Liganden, die befähigt sind, mit in Fluiden enthaltenen Adsorbenden Wechselwirkungen einzugehen, sowie eine Vorrichtung zur hydrophoben Interaktionschromatographie, umfassend mindestens eine erfindungsgemäße Membran.

Die erfindungsgemäße Membran umfaßt eine Cellulosehydrat-Matrix mit Poren, die sich von einer Hauptoberfläche zur anderen Hauptoberfläche der Membran erstrecken, wobei die Membran vernetzt ist und auf ihren inneren und äußeren Oberflächen funktionelle Gruppen in Form von hydrophoben Liganden aufweist.

In einer bevorzugten Ausführungsform der erfindungsgemäßen Membran sind die Mikroporen, die sich von der ersten Hauptoberfläche der Membran durch die Membran zur zweiten Hauptoberfläche erstrecken, unter Ausbildung von Kanälen kommunizierend miteinander verbunden und die Ultraporen erstrecken sich sackförmig von der inneren Oberfläche der Mikroporen in das die Struktur der Membran bildende Material hinein und/oder verbinden benachbarte Mikroporen miteinander.

Unter den Hauptoberflächen sollen die äußeren Flächen der Membran verstanden werden.

Die Porenstruktur der erfindungsgemäßen Membran gibt sich durch die geringe Abhängigkeit des Durchflusses von der Ionenstärke des Mediums als ein Hybrid aus Aerogel und Xerogel ähnlich wie bei vernetzten Agarosegelen zu erkennen. Das steht im Einklang mit der Tatsache, daß auch die Einführung von hydrophoben Liganden zu einer leistungsfähigen Adsorptionsmembran für die hydrophobe Interaktionschromatographie (HIC) führt, wie in den Beispielen der vorliegenden Erfindung gezeigt. Insbesondere hat sich in nicht vorhersehbarer Weise gezeigt, daß die erfindungsgemäße Membran selbst bei hohen Ionenstärken nicht kollabiert und somit die dreidimensionale Struktur erhalten bleibt, wodurch die Trennleistung der Membran deutlich verbessert wird.

Als Ausgangsmaterial für die erfindungsgemäße Adsorptionsmembran dient eine Celluloseester-Membran, die mit mindestens einer Lösung unter Bedingungen in Kontakt gebracht wird, die zum einen zu einer Quellung der Celluloseester-Matrix führen und zum anderen gleichzeitig, d.h. in situ, zur Hydrolyse (Verseifung) der Estergruppen zu Hydroxylgruppen, wobei eine Cellulosehydrat-Membran entsteht.

Die Quellung der Celluloseester-Matrix während der Hydrolyse (Verseifung) der Estergruppen wird durch den Quellungsgrad, d.h. das Verhältnis der Wasserpermeabilität der zuvor mit Wasser benetzten Celluloseester-Membran zur Wasserpermeabilität der endgültigen, d.h. verseiften, aktivierend vernetzten und gegebenenfalls mit Ligand(en) versehenen Cellulosehydrat-Membran, beschrieben.

Anschließend an die Verseifung wird die erhaltene Cellulosehydrat-Matrix durch Umsetzung der Hydroxylgruppen mit einem oder mehreren, mindestens bifunktionellen Reagens vernetzt und danach werden in die vernetzte Matrix funktionelle Gruppen (Liganden) zur Befähigung der adsorptiven Stofftrennung eingeführt.

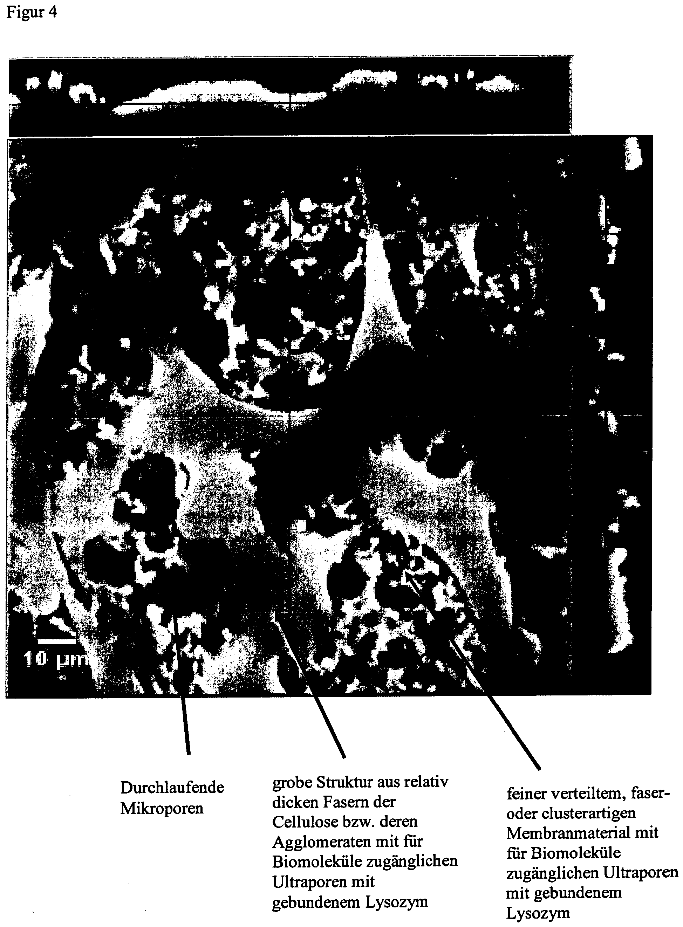

Überraschenderweise wurde festgestellt, daß die Bindungskapazität der Cellulosehydrat-Membran deutlich erhöht wird, wenn der Verseifungsschritt unter Bedingungen durchgeführt wird, unter denen die Cellulose quellen kann. Die Erhöhung der Bindungskapazität für Biomoleküle könnte auf die erhöhte Anzahl an für Biomoleküle zugänglichen, amorphen Bereichen der Cellulose zurückgeführt werden. Durch die Quellung des Celluloseträgers entstehen zwei Arten von Poren: a) Mikroporen mit einem Durchmesser von >100 nm, die in der Regel kleiner als die Originalporen der Celluloseester-Membran sind, und b) Ultraporen (amorphe Bereiche der Cellulose) mit einem Durchmesser von < 100 nm, die dergestalt sind, daß sie für Dextranblau (erhältlich als Blue Dextran molecular weight 2,000,000 von der Firma Sigma, St. Louis, Missouri, USA, Produktnummer D5751, CAS-Nummer: 87915-38-6) nicht zugänglich sind und die eine zusätzliche, für Liganden und Adsorbenden zugängliche Adsorptionsfläche anbieten (vgl. Figur 1c). Die Adsorptionswirksamkeit der erfindungsgemäßen Membran ist nicht auf die Phasengrenzfläche der verbundenen Mikroporen mit dem Medium beschränkt, sondern erstreckt sich zumindest auf einen Teil oder sogar das gesamte Volumen in den Ultraporen des Trägers (siehe Figur 4). Figur 4 zeigt eine konfokale Mikroskopieaufnahme einer mit Lysozym beladenen erfindungsgemäßen Membran mit Ultraporen. Um die unterschiedlichen Einflüsse des Herstellprozesses auf die Eigenschaften der erfindungsgemäßen Membrane besser darstellen und erläutern zu können, wurden neben den hydrophoben Liganden auch ionische Liganden in den Beispielen verwendet.

Die Quellung der Cellulose während der Verseifung kann durch eine geeignete Vorbehandlung des Celluloseesters oder durch die Parameter der Verseifung (Zusammensetzung des Verseifungsmediums, Art des Additivs, Konzentration des Additivs, Verseifungstemperatur) beeinflußt und gesteuert werden. So kann die Permeabilität und Kapazität der Membran eingestellt werden. Die in dem erfindungsgemäßen Verfahren hergestellten, adsorptiven Cellulosehydrat-Membranen zeigen gegenüber den durch auf dem Fachgebiet bekannten Herstellungsverfahren hergestellten Cellulosehydrat-Membranen deutlich höhere Bindungskapazitäten bei vergleichbaren Permeabilitäten.

Wie im Weiteren beschrieben wird, kann das Verfahren zur Herstellung der erfindungsgemäßen Membran in drei Schritten durchgeführt werden, wobei die Einstellung des gewünschten Quellungsgrads, des Durchflusses und der Bindungskapazität sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung der Celluloseester-Matrix hergestellt werden. Hohe Quellungsgrade der Cellulosehydrat-Matrix können gemäß dem erfindungsgemäßen Verfahren durch hohe Konzentration an Alkalimetallhydroxid im Verseifungsmedium, hohe Konzentration an wasserstoffbrückenbrechenden Verbindungen oder niedrige Temperatur des Verseifungsmediums erreicht werden.

Durch die Art des Vernetzungsmittels, die Konzentration des Vernetzungsmittels, die Konzentration des Vernetzungskatalysators, die Vernetzungsdauer, gegebenenfalls die Art und die Konzentration eines inerten organischen Lösungsmittels und/oder die Vernetzungstemperatur kann der Vernetzungsgrad, die Porengröße und die Anzahl an restlichen, aktiven Gruppen, z.B. Epoxid-Gruppen, gesteuert werden. Dadurch kann die für die Bindung der funktionellen Gruppen oft notwendige Aktivierung bereits in dem Vernetzungsschritt stattfinden.

In einem weiteren Schritt werden funktionelle Gruppen, z.B. an die Hydroxylgruppen, der vernetzten Membran gebunden. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Bevorzugt werden funktionelle Gruppen über Epoxid-Gruppen oder AldehydGruppen an die Cellulosemembran gebunden. Die Einführung der Epoxid-Gruppen kann bereits in dem Vernetzungsschritt oder nachträglich stattfinden.

Die Kombinationen der Einflußgrößen (a) der Herstellungsbedingungen der als Ausgangsmaterial verwendeten Celluloseester-Membran, (b) der Bedingungen der gegebenenfalls durchgeführten Vorbehandlung, (c) der Verseifungsbedingungen und (d) der Vernetzungsbedingungen der Celluloseester-Membran, ermöglichen es auch, aus einer Ausgangsmembran mehrere unterschiedliche Endprodukte herzustellen, was produktionstechnisch eine erhebliche Vereinfachung zur Folge hat.

Ausgangsmembran

Die in dem erfindungsgemäßen Verfahren als Ausgangsmembran verwendete Celluloseester-Membran mit einer Porengröße von 0,1 bis 20 $\mu$m, vorzugsweise 0,5 bis 15 $\mu$m und mehr bevorzugt von 1 bis 10 $\mu$m wird durch ein übliches, auf dem Fachgebiet bekanntes Herstellungsverfahren hergestellt. Zur Bestimmung der Porengröße wird ein "Capillary Flow Porometry Test" durchgeführt. Weitere Details sind der Bedienungsanleitung (Capillary Flow Porometer 6.0, CAPWIN Software System, Fa. Porous Materials Inc.) zu entnehmen. Celluloseester-Membranen können aus Cellulosemonoacetat, Cellulosediacetat, Cellulosetriacetat, Cellulosepropionat, Cellulosebutyrat und Celluloseacetobutyrat oder anderen geeigneten Celluloseestern oder Cellulosenitrat, Methylcellulose oder Ethylcellulose, sowie Gemischen davon, aufgebaut sein, wobei Celluloseacetate, insbesondere Cellulosediacetat, bevorzugt sind bzw. ist. Dem Fachmann ist bekannt, daß die Celluloseester-Membran zum Teil auch Hydroxylgruppen neben den Estergruppen enthalten kann.

**Vorbehandlung**

Vor dem Verseifen kann die Celluloseester-Membran in einem geeigneten Medium vorbehandelt werden. Die Temperatur in dem Vorbehandlungsschritt liegt vorzugsweise in einem Bereich von 20 bis 100 °C, wobei eine Temperatur in einem Bereich von etwa 60 °C bis etwa 80 °C besonders bevorzugt ist. Als Vorbehandlungsmedium kann ein Gas, wie beispielsweise Luft, ein organisches Lösungsmittel, wie beispielsweise ein Alkohol, oder ein wässriges Medium verwendet werden, wobei ein wässriges Medium bevorzugt ist. Das Vorbehandlungsmedium enthält vorzugsweise ein oder mehrere Additiv(e), das bzw. die eine gegenüber einem Celluloseester lösende oder weichmachende Wirkung aufweist bzw. aufweisen. Geeignete Additive sind vor allem Säuren, insbesondere Carbonsäuren, wie Essigsäure, und wasserlösliche Weichmacher für Celluloseester, wie Diacetin, Triacetin und Sulfolan. Es ist jedoch vor allem aus wirtschaftlichen Gründen besonders bevorzugt, Essigsäure als Additiv für das Vorbehandlungsmedium zu verwenden, obwohl auch Diacetin und Triacetin ausgezeichnete Ergebnisse liefern, jedoch teurer sind. Die Konzentration des Additivs in dem Vorbehandlungsmedium unterliegt keinen besonderen Beschränkungen.

Die Dauer der Vorbehandlung hat keinen wesentlichen Einfluß auf den Vorbehandlungseffekt, sofern eine Mindesteinwirkdauer angewendet wird, die eine Temperaturangleichung der Celluloseester-Membran in dem Vorbehandlungsmedium und eine Konzentrationsangleichung des gegebenenfalls verwendeten Additivs in der Membran gewährleistet. Die obere Grenze der Einwirkungsdauer des Vorbehandlungsmediums ist durch jene Zeit bestimmt, ab der eine chemische Umsetzung der Celluloseestermembran mit dem Vorbehandlungsmedium, beispielsweise durch Hydrolyse, eintreten könnte. In anderen Worten ausgedrückt, wird die Einwirkungsdauer des Vorbehandlungsmediums derart eingestellt, daß keine (frühzeitige) Hydrolyse oder Verseifung der vorbehandelten Celluloseester-Membran eintritt. Üblicherweise beträgt die Einwirkungsdauer des Vorbehandlungsmediums auf die Celluloseester-Ausgangsmembran zwischen 0,1 Sekunden und 1 Stunde, wobei eine Einwirkungsdauer von 10 Sekunden bis 10 Minuten bevorzugt ist. Das Ausmaß des Vorbehandlungseffekts ist abhängig von der höchsten Temperatur in Verbindung mit der höchsten Konzentration des Additivs, die auf die Celluloseester-Membran einwirken. Wenn also die Abkühlung oder Ausspülung des Additivs über einen längeren Zeitraum erfolgt, ist das ohne Einfluß auf den bereits erreichten Vorbehandlungseffekt. Die Beendigung der Vorbehandlung kann daher durch Ausspülen des Vorbehandlungsadditivs aus der Membran und/oder Absenkung der Temperatur des Vorbehandlungsmediums erfolgen.

## Verseifung

Die gegebenenfalls vorbehandelte Celluloseester-Membran wird mit einem geeigneten Verseifungsmedium verseift, wodurch sich die Cellulosehydrat-Membran unter Quellung der Cellulose-Matrix ausbildet. Je nach der Art des Vorbehandlungsmediums kann die Celluloseester-Membran trocken oder naß im Verseifungsschritt eingesetzt werden.

Durch die Quellung der Cellulose wird die Zugänglichkeit der Hydroxylgruppen für die Anbindung der funktionellen Gruppen und anschließend für die Adsorbenden verbessert. Das Verseifen der Cellulose-Ester-Membran wird in einem wässrigen Medium mit einem pH-Wert von >7, d.h. ein basisches Medium, durchgeführt, wobei das Verseifungsmedium Natrium- oder Lithiumhydroxid enthält. Es können auch Gemische aus Natrium- oder Lithiumhydroxid und anderen alkalischen Verbindungen wie Alkalimetallcarbonat, wie Natriumcarbonat, Kaliumcarbonat, Cäsiumcarbonat, Natriumhydrogencarbonat, und/oder Natriumtriphosphat, Kaliumtriphosphat, Natriumsilikat und Kaliumsilicat eingesetzt werden.

Die Konzentration des Natrium- oder Lithiumhydroxids im Verseifungsmedium beträgt 0,4 bis 50 Gew.-%, bezogen auf das Verseifungsmedium, und besonders bevorzugt 0,4 bis 10 Gew.-%. Gemäß einer besonders bevorzugten Ausführungsform der vorliegenden Erfindung wird ein Verseifungsmedium aus Wasser und Natriumhydroxid verwendet, wobei die Konzentration des Natriumhydroxids in dem Verseifungsmedium in einem Bereich von 0,4 bis 20 Gew.-%, besonders bevorzugt in einem Bereich von 0,4 bis 4 Gew.-% liegt.

Das Verseifungsmedium kann ein oder mehrere Additiv(e) enthalten, das bzw. die eine gegenüber einem Celluloseester quellungsbeeinflussende Wirkung aufweist bzw. aufweisen. Geeignete Additive sind vor allem Salze, wie Natriumchlorid, Natriumsulfat und Natriumacetat, Wasserstoffbrücken brechende Verbindungen, wie Harnstoff oder organische Lösungsmittel, wie Ethylamin. Das organische Lösungsmittel wird vorzugsweise aus der Gruppe von Alkohlen, Ketonen oder Ethern ausgewählt. Besonders bevorzugt ist Ethanol, Methanol, Ethylenglycol, Propylenglycol, Glycerin, Aceton, Dioxan oder Diglyme. Das Additiv in dem Verseifungsmedium sollte die Quellung beeinflussen, aber nicht vollständig unterdrücken.

Die Temperatur des verwendeten Mediums in dem Verseifungsschritt kann von etwa 10 °C bis zum Siedepunkt des Verseifungsmediums betragen, wobei eine Temperatur in einem Bereich von 15 °C bis etwa 25 °C bevorzugt ist.

Die Verseifungsdauer richtet sich nach der Zusammensetzung des Verseifungsmediums und der Verseifungstemperatur. Üblicherweise beträgt die Verseifungsdauer 0,1 bis 60 Minuten, wobei eine Verseifungsdauer von 5 bis 45 Minuten bevorzugt ist. Besonders bevorzugt ist eine Verseifungsdauer von 20 bis 40 Minuten.

## Vernetzung

Die nach der gegebenenfalls durchgeführten Vorbehandlung und der unter Quellung stattgefundenen Verseifung erhaltene Cellulosehydrat-Membran wird mit einem Vernetzungsmittel zur Erhöhung der chemischen Widerstandsfähigkeit der Membran und/oder zur Einführung von funktionellen Gruppen vernetzt.

Das Vernetzungsmittel weist mindestens zwei funktionelle Gruppen im Molekül auf, die mit den Hydroxylgruppen von Cellulose reaktiv sind und somit eine Vernetzung von Cellulose ermöglichen. Die verwendbaren Vernetzungsmittel unterliegen grundsätzlich keinen besonderen Beschränkungen und ein Fachmann ist in der Lage, sie aus einer Reihe von für die Vernetzung von Cellulose verwendbaren Vernetzungsmitteln auszuwählen. Es ist jedoch bevorzugt, in dem Vernetzungsschritt eine Diepoxidverbindung oder auch andere, mit Hydroxylgruppen von Cellulose reaktive Verbindungen mit mindestens zwei reaktiven, funktionellen Gruppen, wie Diisocyanat, Epichlorhydrin, Epibromhydrin, Dimethylharnstoff, Dimethylethylenharnstoff, Dimethylchlorsilan, Bis(2-hydroxyethylsulfon), Divinylsulfon, Alkylen-Dihalogen, Hydroxyalkylen-Dihalogen und Glycidylether zu verwenden.

Aus der Gruppe der Glycidylether sind 1,4-Butandioldiglycidylether, Ethylenglycoldiglycidylether, Glycerindiglycidylether und Polyethylenglycoldiglycidylether bevorzugt.

Besonders bevorzugt ist die Verwendung von 1,4-Butandioldiglycidylether oder Epichlorhydrin als Vernetzungsmittel.

Optional kann ein Gemisch von unterschiedlichen Vernetzungsmitteln verwendet werden.

Die Vernetzung kann in einem wässrigen Medium, in einem organischen Lösungsmittel oder auch in einem Gemisch aus Wasser und einem organischen Lösungsmittel stattfinden. Vorzugsweise wird die Vernetzung in einem wässrigen Medium durchgeführt.

Ferner ist es bevorzugt, einen Vernetzungskatalysator, wie Natriumhydroxid, zur Beschleunigung der Vernetzung von Cellulose mit dem Vernetzungsmittel zu verwenden.

Die Temperatur des verwendeten Mediums in dem Vernetzungsschritt kann von etwa 4 °C bis zum Siedepunkt des Vernetzungsmediums betragen, wobei eine Temperatur in einem Bereich von 5 °C bis etwa 70 °C bevorzugt ist. Besonders bevorzugt ist eine Temperatur von 20 °C bis 40 °C.

Üblicherweise beträgt die Vernetzungsdauer 10 Minuten bis 100 Stunden, wobei eine Verseifungsdauer von 30 Minuten bis 48 Stunden bevorzugt ist. Besonders bevorzugt ist eine Verseifungsdauer von 2 bis 24 Stunden.

Wie vorstehend beschrieben, kann das Verfahren zur Herstellung der erfindungsgemäßen Membran in drei Schritten durchgeführt werden, wobei die Einstellung des gewünschten Quellungsgrads der Matrix sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung hergestellt werden.

**Aktivierung und Bindung von funktionellen Gruppen**

In einem weiteren Schritt werden funktionelle Gruppen, d.h. hydrophobe Liganden an die Hydroxylgruppen der vernetzten Cellulosehydrat-Membran gebunden. Methoden zur Bindung von funktionellen Gruppen sind dem Fachmann bekannt (z.B. aus Greg T. Hermanson, A. Krishna Mallia, Paul K. Smith, Immobilized Affinity Ligand Techniques, Academic Press, INC, 1992).

Ein übliches Verfahren zur Einführung von funktionellen Gruppen beruht auf der Umsetzung der mit Oxirangruppen aktivierten Membran mit einem Alkohol in Gegenwart von Bortrifluoridetherat. Andere Herstellverfahren beruhen auf der Umsetzung eines hydroxylgruppenhaltigen Trägers mit einem oxirangruppenhaltigen Molekül in Gegenwart von Bortrifluoridetherat. Andere Verfahren basieren auf der Umsetzung von oxiranhaltigen aktivierten Trägern mit Aminen.

Bevorzugt werden die hydrophoben Liganden über Epoxid-Gruppen oder AldehydGruppen an die Cellulosemembran gebunden. Die Epoxid-Aktivierung kann bereits in dem Vernetzungsschritt oder nachträglich stattfinden.

Es ist auch möglich, die hydrophoben Liganden während der Vernetzung einzuführen, z.B. durch Zugabe von einem Amin und/oder von einer monofunktionellen Epoxidverbindung, wie Phenylglycidylether oder Butylglycidylether zu einer Diepoxidverbindung.

Die funktionellen Gruppen für die erfindungsgemäße Membran sind ausgewählt aus: C1-C20-Alkyl und deren Derivate oder C6-C25-Aryl und deren Derivate oder C7-C25-Arylalkyl und deren Derivate oder-[(CH2)m-O-]n-R, wobei m 2 oder 3 ist, n eine ganze Zahl von größer als 1 ist, und wobei R -H oder -C1-C5-Alkyl bedeuten.

Die erfindungsgemäßen Membranen können nach der Einführung der Liganden optional getrocknet werden. Membranen können direkt aus Wasser oder organischen Lösungsmitteln, bevorzugt Alkohol, getrocknet werden, oder nach einem stufenweise durchgeführten Austausch des Wassers durch ein organisches Lösungsmittel. Bevorzugt werden die Membranen aus einem Medium getrocknet, das eine porenstabilisierende Verbindung enthält. Besonders bevorzugt werden die erfindungsgemäßen Membranen aus wässriger Glycerinlösung getrocknet. Die Konzentration des Glycerins liegt vorzugsweise im Bereich von 5 bis 40 Gewichtsprozent, bezogen auf die wässrige Lösung.

**Erläuterung der Beispiele**

Vernetzte Cellulosehydrat-Membranen mit niedrigem Quellungsgrad werden beispielsweise aus mit ethanolischer Kalilauge verseiften Celluloseester-Membranen hergestellt. Eine Celluloseacetat-Membran ergibt auf diese Weise eine vernetzte Cellulosehydrat-Membran mit geringfügig niedrigerem Durchfluss (siehe Beispiel 1), die aber nach Einführung von Liganden praktisch kein Adsorptionsvermögen (Bindungskapazität) aufweist (siehe Tabelle 5).

Es wurde nun gefunden, daß bei Verseifung mit wässriger Natronlauge zwar der Durchfluß sinkt (siehe Beispiel 2), nach Belegung mit diversen Liganden aber deutlich erhöhte Bindungskapazitäten auftreten, wobei steigende NatronlaugeKonzentrationen sich in Richtung einer stärkeren Durchflußminderung und höheren Bindungskapazitäten auswirken (siehe Tabelle 5). Gegenüber den durchgehenden Mikroporen, die sich bei der Verseifung mit ethanolischer Kalilauge vorwiegend bilden, scheint bei der Verseifung mit wässriger Natronlauge die Entstehung einer Vielzahl kleiner Ultraporen bevorzugt zu sein. Eine höhere Verseifungstemperatur sowie ein zusätzlicher Gehalt an Elektrolyten einschließlich bei der Verseifung bereits gebildeten Natriumacetats wirken sich dabei in der gleichen Richtung aus wie eine niedrigere Natronlauge-Konzentration.

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Durch gleichzeitige Verseifung und Vernetzung unter Bedingungen, die die Quellung und Strukturänderung unterdrücken ($Na_2SO_4$, niedrige Natronlauge-Konzentration) wird keine signifikante Bindungskapazität festgestellt (siehe Vergleichsbeispiel 1). Erst wenn die Laugenkonzentration erhöht wird, kommt es zur Erhöhung der Bindungskapazität. Durch die Quellung der Cellulose kommt es hier aber auch zur Änderung der Porenstruktur, was im Widerspruch zum in dem Stand der Technik beschriebenen, simultanen Verseifungs- und Vernetzungsprozess steht. Die Bindungskapazität beträgt hier aber nur ca. 5 % der Bindungskapazität im Vergleich zu der separat durchgeführten Verseifung und Vernetzung (siehe Beispiel 2 und Vergleichsbeispiel 1).

Weiterhin wurde gefunden, daß sich die unterschiedliche Vorbehandlung der Celluloseacetat-Membran unterschiedlich auf die Eigenschaften der erfindungsgemäßen adsorptiven Membran auswirkt. Der Durchfluss sinkt und die Bindungskapazität steigt, wenn die Celluloseacetat-Membran vor der Verseifung auf 80 °C an der Luft erhitzt wurde (siehe Beispiel 3). Wenn die Celluloseacetatmembran vor der Verseifung in 20%iger Essigsäure auf 80 °C erhitzt wird (siehe Beispiel 4), steigt der Durchfluss im Vergleich zu der nicht vorbehandelten Membran aus dem Beispiel 2 und die Bindungskapazität ändert sich abhängig von der Größe des Proteins. Für Lysozym (14,3 kDa) steigt die Bindungskapazität, für Rinderserumalbumin (RSA; 60 kDa) und Gamma-Globulin (150 kDa) sinkt die Bindungskapazität. Die Vorbehandlung kann beispielsweise bei speziellen Trennaufgaben dann vorteilhaft sein, wenn die Spezifität des Liganden für die Stofftrennung alleine nicht ausreicht und die Molmassen der zu trennenden Komponenten so stark unterschiedlich sind, daß sich aus der Überlagerung der rein adsorptiven Stofftrennung mit einem Größenausschlußeffekt insgesamt eine Verbesserung der Trennleistung ergibt, und die Beeinflussung der Porengröße der Ultraporen durch die Wahl der Base und deren Konzentration einer Unterstützung bedarf. Eine vollständige Trennung auf der Basis allein dieses Effekts ist allerdings nicht möglich, weil der Größenausschluß nur für die Adsorption an der inneren Oberfläche der Ultraporen wirksam wird, nicht aber an der äußeren Oberfläche der Mikroporen.

Diese Befunde lassen bei der Verseifung und Vernetzung von Celluloseacetaten auf komplexe Quellungs- und Entquellungsvorgänge schließen, deren Auswirkungen im Hinblick auf die Struktur des Endprodukts schwer zusammenfassend darzustellen sind, weil es sowohl Vorgänge gibt, bei denen eine Durchflussminderung mit einer Erhöhung der Bindungskapazität gekoppelt ist, als auch solche, bei denen das nicht der Fall ist. Die erstgenannten werden im folgenden als "produktiv" bezeichnet, die sonstigen als "unproduktiv". Die Vorbehandlung der Celluloseacetat-Membran wirkt sich unterschiedlich auf die Änderung der Porenstruktur, die Bildung von Mikroporen sowie von Ultraporen aus. So ist es möglich, durch geeignete Wahl der Vorbehandlung den Durchfluss, die Bindungskapazität aber auch den Größenausschluss der erfindungsgemäßen adsorptiven Membran zu beeinflussen. Das Hauptziel des erfindungsgemäßen Verfahrens ist die Beschränkung auf produktive Durchflussminderungen, wobei nicht nur das Quellverhalten des Ausgangsmaterials, der Celluloseacetat-Membran, und des Endprodukts, der vernetzten Cellulosehydrat-Membran, in Betracht zu ziehen sind, sondern auch das gesamte Spektrum der Zwischenprodukte im teilverseiften und teilvernetzten Zustand. So ist beispielsweise bekannt, daß Celluloseacetate abnehmenden Acetylgehalts in einem engen Bereich sogar einen Zustand der Wasserlöslichkeit durchlaufen.

Erfindungsgemäß wird eine Celluloseester-Membran sequentiell in einem quellenden Medium verseift, mit einem mindestens bifunktionellen Agens vernetzt und mit einem adsorptionswirksamen Liganden versehen. Das Quellvermögen der Alkalimetallhydroxide nimmt in Richtung kleinerer Kationenradien und höherer Konzentrationen zu (siehe Beispiel 5).

Die Vernetzung der Cellulose erfolgt erfindungsgemäß vorzugsweise mit 1,4-Butandioldiglycidylether. In einer Ausgestaltung der Erfindung erfolgt die Vernetzung der Cellulose mit 1,4-Butandioldiglycidylether in der Weise, dass infolge einer zum Teil einseitigen Reaktion eine ausreichende Zahl nicht umgesetzter Epoxidgruppen erhalten bleibt ("aktivierende Vernetzung", siehe Beispiel 2), die zur Bindung oder zur Ankopplung bzw. zum Aufbau von Liganden dienen können. Die nicht umgesetzten Epoxidgruppen sind relativ hydrolysestabil und wurden noch nach bis zu 24 Stunden Feuchtlagerung bei Raumtemperatur für Folgereaktionen eingesetzt. In einer anderen Ausführungsform der Erfindung zur Bindung "aktiver" Liganden wird die Vernetzung unter verschärften Bedingungen (längere Vernetzungsdauer und/oder höhere Alkalikonzentration und/oder höhere Temperatur) durchgeführt, so dass unter gesteigerter Reaktion mit der Cellulose und/oder gesteigerter Hydrolyse der überzähligen Gruppen ein Verbleib von Epoxidgruppen weitgehend unterbleibt ("nicht aktivierende Vernetzung", siehe Beispiel 6). Restliche Epoxidgruppen können auch durch nachträgliche Behandlung mit z.B. 5%iger Schwefelsäure bei erhöhter Temperatur hydrolisiert werden.

Der Durchfluß der Membran nach Beispiel 2 für 20 mM tris/HCl Puffer mit einem pH-Wert von 7,3 ist um 8% größer als der für reines Wasser.

Entsprechende Werte von Adsorptionsmembranen, die nach dem Stand der Technik durch Beschichtung hergestellt wurden, liegen zwischen 20 % im Fall eines vernetzten Hilfspolymeren und 200 % bei einem unvernetzten. Die entstandene Porenstruktur gibt sich durch die geringe Abhängigkeit des Durchflusses von der Ionenstärke des Mediums als ein Hybrid aus Aerogel und Xerogel ähnlich wie bei einem vernetzten Agarosegel zu erkennen. Das steht im Einklang mit der Tatsache, daß auch die Einführung von hydrophoben Liganden zu einer leistungsfähigen Adsorptionsmembran für die hydrophobe Interaktionschromatographie (HIC) führt (siehe Beispiele 4 und 9).

Die erfindungsgemäßen Adsorptionsmembranen lassen sich von nach dem Stand der Technik durch Polymerbeschichtung oder Pfropfung hergestellten Adsorptionsmembranen durch Rasterelektronenmikroskopie schwer unterscheiden, weil deren Auflösungsvermögen durch die den Hauptunterschied ausmachenden kleinporigen Strukturen (d.h. Ultraporen) überfordert wäre. Dagegen liefert die Charakterisierung von adsorptiven Membranen mittels der sogenannten konfokalen Laserfluoreszenzmikroskopie (CLSM) unter geeigneten Bedingungen simultan sowohl Information zur Porenstruktur als auch zur Verteilung von an funktionellen Gruppen gebundenem Protein in der Membran. Dafür müssen Membranmaterial und Protein mit zwei verschiedenen Fluoreszenzfarbstoffen markiert werden. Alle mikroskopischen Messungen wurden ungefähr im selben Abstand (ca. 20 $\mu$m) von der jeweiligen äußeren Oberfläche durchgeführt. In allen Fällen führten drei unabhängige Messungen an verschiedenen x, y-Positionen zu sehr ähnlichen, für den jeweiligen Membrantyp charakteristischen Ergebnissen.

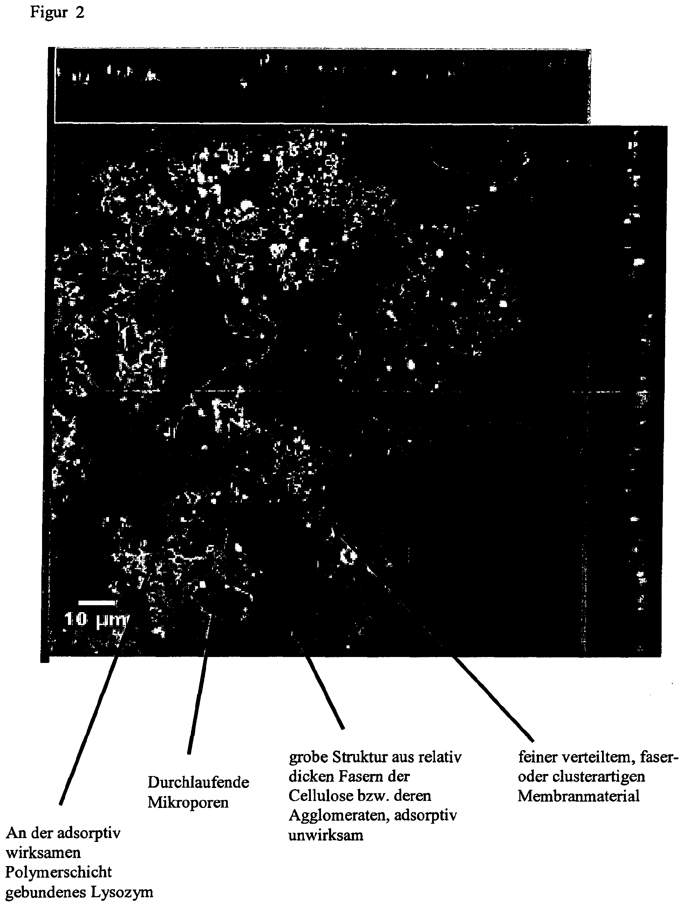

Charakteristisch für alle Membranproben ist eine sehr grobe Struktur (dunkle Bereiche in den Figuren 2 - 4) aus relativ dicken Fasern bzw. deren Agglomeraten mit feiner verteiltem Faser- oder clusterartigen Membranmaterial mit dazwischen sich befindlichen, vollständig oder teilweise ungefärbten Bereichen, die den durchlaufenden Mikroporen mit Dimensionen bis zu ca. 20 $\mu$m zugeordnet werden können. Die Proteinverteilung war für alle Membranproben eindeutig zu identifizieren. Allerdings wurden sehr große Unterschiede in Bezug auf Proteinmenge (Fluoreszenzintensität, helle Bereiche) und Proteinverteilung in der Porenstruktur (dunkle Bereiche) gefunden. Die Gesamt-Fluoreszenzintensitäten waren deutlich unterschiedlich, für die Membran nach Beispiel 2 mußte sogar eine geringere Verstärkung als für die anderen Membranen gewählt werden:

Membran aus Beispiel 2 > Sartobind® S Membran >> Membran aus Beispiel 1

Diese Ergebnisse korrelieren gut mit den Angaben zur Bindungskapazität:

Membran nach Beispiel 1: 0,01 mg/cm$^2$
Membran Sartobind® S: 0,90 mg/cm$^2$
Membran nach Beispiel 2: 2,06 mg/cm$^2$

Mit der Untersuchungsmethode konnten eindeutige und große Unterschiede in Bezug auf die Proteinbindung zwischen der etablierten Sartobind® S Membran (Figur 2) sowie den aus dem Beispiel 1 (Figur 3) und dem Beispiel 2 (Figur 4) mit Sulfonsäureliganden funktionalisierten Membranen identifiziert werden. Bei den Membranen aus den Beispielen 1 und 2 korrelieren die Fluoreszenzintensitäten für das Protein (helle Bereiche) mit den nominalen Proteinbindungskapazitäten, d.h. die Membran aus dem Beispiel 1 weist eine nur sehr geringe Bindungskapazität auf, wobei die Membran aus dem Beispiel 2 eine deutlich höhere Bindungskapazität aufweist.

Ausgehend von derselben Porenstruktur des Basisträgers bindet das Protein in den Sartobind® S Membranen vor allem im Volumen der Mikroporen, wobei wesentlich für die Proteinbindung eine dreidimensionale funktionelle Schicht ist. Dabei zeigen diese Membranen an den Rändern der Poren scharfe Abgrenzungen zwischen dem Material der Membran (dunkle Bereiche) und der Proteinschicht (helle Bereiche). Wegen der begrenzten Reichweite dieser funktionellen Schicht verbleiben kleine Anteile am Porenvolumen, in denen kein Protein gebunden ist. Bei der Membran aus dem Beispiel 1 findet die Bindung direkt am Membranmaterial statt, was durch kleine helle Punkte in der Figur 2 zu erkennen ist. Im Gegensatz dazu werden bei der Membran aus dem Beispiel 2 offensichtlich sehr große Mengen an Protein in den Ultraporen der groben Faserstruktur sowie in dem feiner verteilten, faser- oder clusterartigen Membranmaterial gebunden. Zwischen den Verteilungen von Cellulose und Protein wird eine sehr gute Korrelation gefunden, visuell auch daran erkennbar, daß in der Überlagerung nur die Mischfarbe der verwendeten Farbstoffe zu erkennen ist, weil in der Tiefe der Ultraporen sowohl Porenoberfläche als auch Protein sichtbar sind. Der weitaus größte Anteil des Volumens der Mikroporen enthält kein Protein.

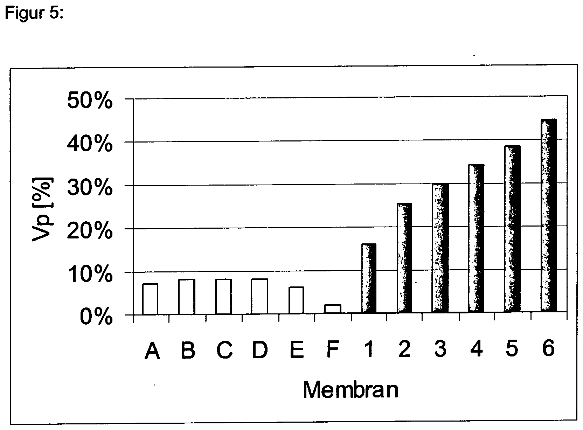

Um die Ultraporen der erfindungsgemäßen Membranen quantitativ zu erfassen, wurde ein Versuch durchgeführt, in dem die Porenzugänglichkeit für Dextranblau bestimmt wurde. Die Versuchsdurchführung erfolgte in der in Beispiel 14 beschriebenen Weise. Das Ergebnis der Auswertung ist in Tabelle 1 und in Figur 5 gezeigt.

In der Figur 5 ist ein deutlicher Unterschied zwischen den für Dextranblau unzugänglichen Ultraporen für die im Stand der Technik bekannten Membranen A-F und Membranen aus dem Beispiel 14 zu erkennen. Im Falle der Membranen, die unter quellenden Bedingungen verseift wurden, liegt mehr als 15 % des gesamten Porenvolumens im Bereich von Ultraporen (d.h. Poren mit einem Durchmesser <100 nm, die für Dextranblau nicht zugänglich sind), wohingegen es bei den Vergleichsmembranen A-F weniger als 8 % sind.

Demgemäß weisen erfindungsgemäße Membranen ein Volumen an Ultraporen, die zwar für Wasser zugänglich sind, jedoch nicht für Dextranblau mit einem Molekulargewicht Mw von 2.000.000, von mehr als 15 %, vorzugsweise mehr als 18 %, mehr bevorzugt von mehr als 20 %, noch mehr bevorzugt von mehr als 25 % und am meisten bevorzugt von mehr als 30 % des gesamten Porenvolumens auf.

**Figuren**

Figur 1a): Schematische Darstellung der Proteinbindung an Mikroporen einer wie im Beispiel 1 hergestellten und im Stand der Technik bekannten adsorptiven Membran.

Figur 1b): Schematische Darstellung der Proteinbindung an einer adsorptiv wirksamen Polymerbeschichtung von wie in dem Stand der Technik beschriebenen adsorptiven Membranen, die aus einer oder mehreren Trägerstruktur(en) und einer oder mehreren adsorptiv wirksamen Polymerbeschichtung(en) bestehen.

Figur 1c): Schematische Darstellung der Proteinbindung in den Ultraporen einer erfindungsgemäßen adsorptiven Membran.

Figur 2: CLSM-Bild der Porenmorphologie und Proteinverteilung an der Oberseite der Sartobind® S Membran nach Markierung der Cellulose mit Fluoreszenzfarbstoff und Beladung mit Fluoreszenz-markiertem Lysozym.

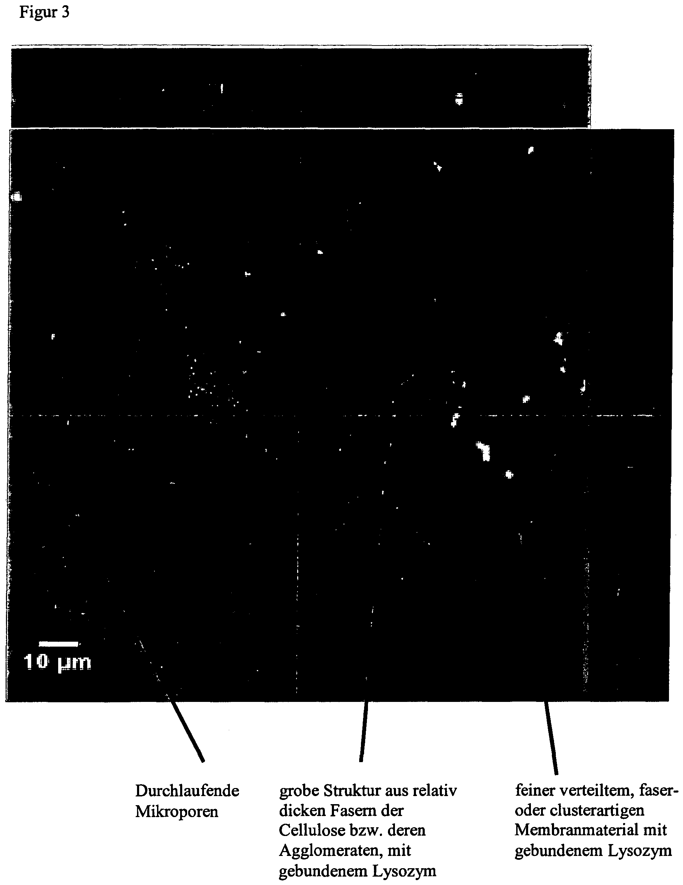

Figur 3: CLSM-Bild der Porenmorphologie und Proteinverteilung an der Oberseite der mit Sulfonsäureliganden umgesetzten Membran nach Beispiel 1 nach Markierung der Cellulose mit Fluoreszenzfarbstoff und Beladung mit Fluoreszenz-markiertem Lysozym.

Figur 4: CLSM-Bild der Porenmorphologie und Proteinverteilung an der Oberseite der mit Sulfonsäureliganden umgesetzten Membran nach Beispiel 2 nach Markierung der Cellulose mit Fluoreszenzfarbstoff und Beladung mit Fluoreszenz-markiertem Lysozym.

Figur 5: Vergleich des prozentualen Anteils von für Dextranblau mit einem Molekulargewicht Mw von 2.000.000 unzugänglichen Poren in den Membranen:

A: Membrane aus Beispiel 1
B-F: Cellulose-Membranen nach dem Stand der Technik 0,2-0,45 $\mu$m der Fa. Sartorius Stedim Biotech GmbH
1-6: Membranen aus dem Beispiel 14

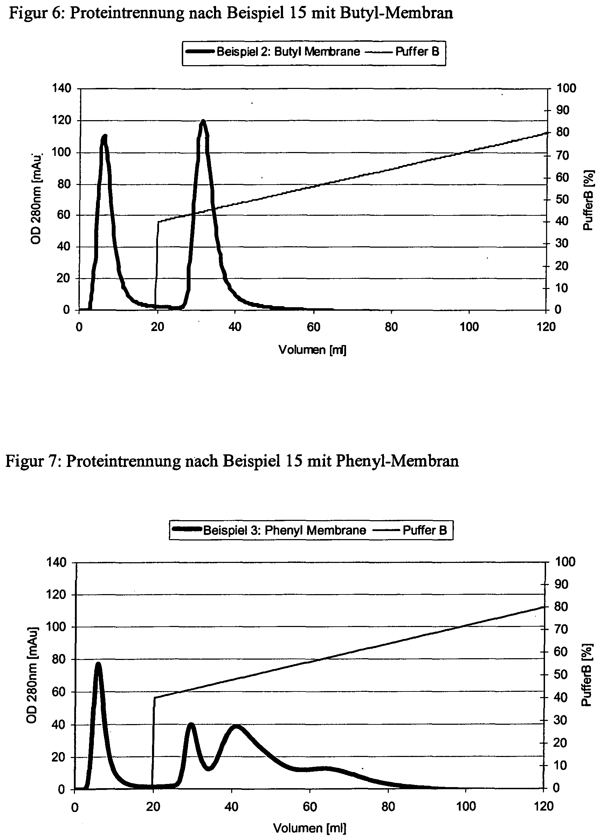

Figur 6: Proteintrennung nach Beispiel 15 mit einer Membran mit Butylliganden (Butyl-Membran)

Figur 7: Proteintrennung nach Beispiel 15 mit einer Membran mit Phenylliganden (Phenyl-Membran)

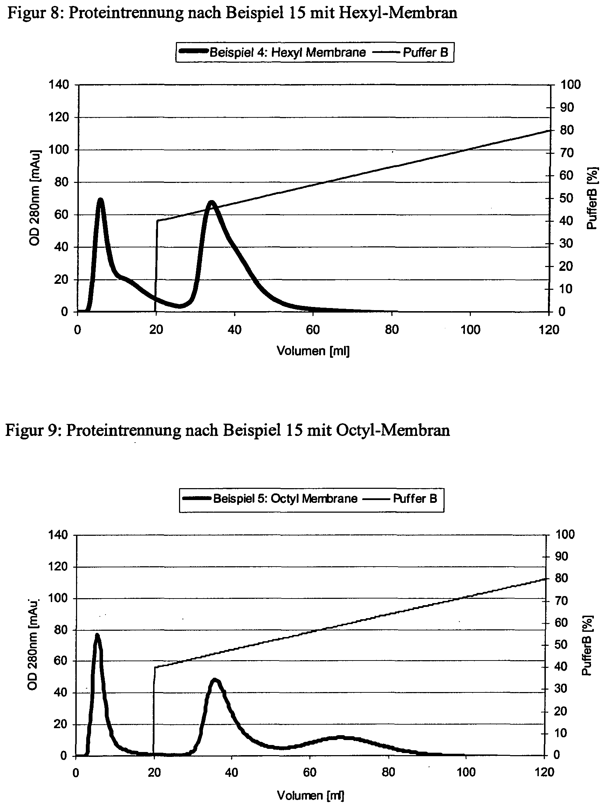

Figur 8: Proteintrennung nach Beispiel 15 mit einer Membran mit Hexylliganden (Hexyl-Membran)

Figur 9: Proteintrennung nach Beispiel 15 mit einer Membran mit Octylliganden (Octyl-Membran)

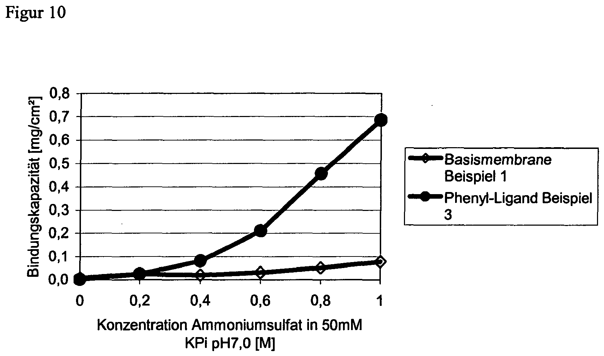

Figur 10: Abhängigkeit der Bindungskapazität einer wie in Beispiel 9 hergestellten Membran mit Phenylliganden (Phenyl-Membran) von der Ammoniumsulfatkonzentration im Filtermedium.

**Beispiele**

Sämtliche Erwähnungen in den Beispielen einer CA-Membran beziehen sich auf einen mit Polyester-Vlies verstärkten Typ einer Celluloseacetat-Membran mit einem Porendurchmesser von ca. 3 $\mu$m (gemessen mit einem Coulter-Porometer Typ Capillary Flow Porometer 6.0, CAPWIN Software System, Fa. Porous Materials Inc.), welche einen Wasserdurchfluss von 730 ml/(min x bar x cm$^2$) aufweist. Die Dicke der modifizierten Membranproben betrug durchschnittlich 250 $\mu$m. Alle Durchflussangaben sind in ml/(min x bar x cm$^2$) und alle Bindungskapazitätsangaben sind in mg/cm$^2$. Sofern nichts anderes angegeben ist, beziehen sich Prozentangaben auf das Gewicht.

**Beispiel 1**

Aktivierend vernetzte Cellulosehydrat-Membran mit einem niedrigen Quellungsgrad für Vergleichsbeispiele

Die Membran wurde in folgender Weise hergestellt: Als Ausgangsmaterial wurde eine wie vorstehend genannte CA-Membran verwendet. Diese CA-Membran wurde drei Minuten bei Raumtemperatur mit 15%iger Kalilauge-Lösung in 80%igem Ethanol verseift. Anschließend wurde drei Minuten mit einer 6,8%igen Essigsäurelösung, zweimal mit Ethanol und danach 15 Minuten mit fließendem RO-(d.h. reverse osmosis)-Wasser gespült. Danach wurde die Membran 20 Minuten bei 80°C im Umlufttrockenschrank getrocknet.

Im nächsten Schritt wurde die so erhaltene, getrocknete Membran 30 Minuten bei Raumtemperatur mit 30%igem 1,4-Butandioldiglycidylether in wässriger 0,1 M Natronlauge-Lösung und wässriger 0,1%iger Natriumborhydrid-Lösung behandelt, und dann die feuchte Membran 20 Stunden in einem abgeschlossenen Gefäß bei Raumtemperatur stehen gelassen.

Abschließend wurde die Membran 30 Minuten mit fließendem Wasser gespült.

Der Wasserdurchfluß der so hergestellten, aktivierend vernetzten Cellulosehydrat-Membran betrug 630 ml/(min x bar x $cm^2$). Der Quellungsgrad betrug 1,16.

**Beispiel 2**

Aktivierend vernetztes Zwischenprodukt für eine erfindungsgemäße Adsorptionsmembran

Als Ausgangsmaterial wurde eine CA-Membran wie in Beispiel 1 verwendet. Diese CA-Membran wurde 30 Minuten bei Raumtemperatur mit 0,6 M wässriger Natronlauge-Lösung (d.h. unter quellenden Bedingungen) verseift und anschließend 3 x 10 Minuten mit 0,5 M wässriger Natronlauge-Lösung gespült. Die erhaltene Membran wurde 30 Minuten bei Raumtemperatur mit 30%igem 1,4-Butandioldiglycidylether in 0,1 M wässriger Natronlauge-Lösung und 0,1%iger, wässriger Natriumborhydrid-Lösung behandelt (d.h. vernetzt), und danach wurde die feuchte Membran 20 Stunden in einem abgeschlossenen Gefäß bei Raumtemperatur stehengelassen.

Abschließend wurde 30 Minuten mit fließendem Wasser gespült.

Der Wasserdurchfluss des aktivierend vernetzten Zwischenprodukts betrug 45 ml/(min x bar x $cm^2$) und der Quellungsgrad betrug 16,2.

**Beispiel 3**

Aktivierend vernetztes Zwischenprodukt für eine erfindungsgemäße Adsorptionsmembran: Vorbehandlung der CA-Membran

Eine CA-Membran wurde in gleicher Weise behandelt wie in Beispiel 2, mit der Ausnahme, daß die CA-Membran vor der Verseifung 20 Minuten bei 80 °C im Trockenschrank erhitzt wurde.

Der Wasserdurchfluss des resultierenden, aktivierend vernetzten Zwischenprodukts betrug 21 ml/(min x bar x $cm^2$) und der Quellungsgrad betrug 34,8.

**Beispiel 4**

Aktivierend vernetztes Zwischenprodukt für eine erfindungsgemäße Adsorptionsmembran: Vorbehandlung der CA-Membran

Eine CA-Membran wurde in gleicher Weise behandelt wie in Beispiel 2 mit der Ausnahme, daß die CA-Membran vor der Verseifung in 20%iger Essigsäure-Lösung auf 80 °C erhitzt wurde und 15 Minuten mit fließendem Wasser gespült wurde.

Der Wasserdurchfluss des resultierenden, aktivierend vernetzten Zwischenprodukts betrug 180 ml/(min x bar x $cm^2$) und der Quellungsgrad betrug 4,06.

**Beispiel 5**

Verschiedene Alkalihydroxide

CA-Membranen wurden jeweils in 0,5 M wässrigen Lösungen von LiOH, NaOH und KOH 30 Minuten bei Raumtemperatur verseift, anschließend ohne Spülung mit wässrigen Lösungen von 15%igem 1,4-Butandioldiglycidylether und 0,1%iger Natriumborhydrid-Lösung in den gleichen Alkalihydroxidlösungen bei Raumtemperatur 3,5 Stunden vernetzt. Die Membranen wurden weiter mit einem quaternären Ammonium-Liganden umgesetzt, in dem die vernetzten Membranen 35 Minuten bei 30 °C in einer 10%igen, wässrigen Lösung von Trimethylamin und 5 Minuten bei Raumtemperatur in 5%iger Schwefelsäure-Lösung behandelt und danach 10 Minuten mit fließendem Wasser gespült wurden. Die Ergebnisse sind in der Tabelle 5 angegeben.

**Beispiel 6**

Nichtaktivierend vernetztes Zwischenprodukt für erfindungsgemäße Adsorptionsmembran

Als Ausgangsmembran wurde eine CA-Membran wie in Beispiel 1 verwendet. Diese CA-Membran wurde 30 Minuten bei Raumtemperatur mit 0,6 M wässriger Natronlauge-Lösung verseift und anschließend 3 x 10 Minuten mit 0,5 M wässriger Natronlauge-Lösung gespült. Die erhaltene Membran wurde 30 Minuten bei Raumtemperatur mit wässrigem, 15%igen 1,4-Butandioldiglycidylether in 0,5 M wässriger Natronlauge-Lösung und 0,1%iger, wässriger Natriumborhydridlösung behandelt (vernetzt) und danach wurde die feuchte Membran 20 Stunden in einem abgeschlossenen Gefäß bei Raumtemperatur stehengelassen. Abschließend wurde 30 Minuten mit fließendem Wasser gespült.
Der Wasserdurchfluss des nicht-aktivierend vernetzten Zwischenprodukts betrug 31 ml/(min x bar x cm$^2$) und der Quellungsgrad betrug 23,5.

**Beispiel 7**

Einführung von quaternären Ammonium-Liganden (Q-Membran)

Aktivierend vernetzte Membranen (Zwischenprodukte) wurden 35 Minuten bei 30 °C in einer 10%eigen; wässrigen Lösung von Trimethylamin und 5 Minuten bei Raumtemperatur in 5%iger Schwefelsäure-Lösung behandelt und danach 10 Minuten mit fließendem Wasser gespült, wodurch Membranen mit quaternären Ammonium-Liganden (im folgenden: Q-Membranen) erhalten wurden.

**Beispiel 8**

Einführung von Sulfonsäure-Liganden (S-Membran)

Aktivierend vernetzte Membranen (Zwischenprodukte) wurden 45 Minuten bei 80 °C in einer wässrigen Lösung von 30 % Natriumsulfit und 2,5 % $Na_2HPO_4$ x $H_2O$ bei einem pH-Wert von 8,0 behandelt und danach 10 Minuten mit fließendem Wasser, 5 Minuten mit 35 g einer 1%igen HCl-Lösung, 2 x 5 min mit je 30 g einer wässrigen 1 M NaCl-Lösung, 5 min mit 500 g 5%iger $H_2SO_4$-Lösung und 10 Minuten mit fließendem Wasser gespült, wodurch Membranen mit Sulfonsäure-Liganden (S-Membranen) erhalten wurden.

**Beispiel 9**

Einführung von Phenyl-Liganden (Ph-Membran)

Aktivierend vernetzte Membranen (Zwischenprodukte) wurden drei Stunden bei Raumtemperatur mit einer wässrigen Lösung von 1 % Anilin in 0,1 M Kpi (Kaliumphosphat)-Puffer bei einem pH-Wert von 7,8 behandelt und die feuchten Proben wurden 19 Stunden im verschlossenen Gefäß belassen. Nach 15 Minuten Spülen mit fließendem Wasser wurde 15 Minuten mit 1 M wässriger NaCl-Lösung und 15 Minuten mit fließendem Wasser nachgespült, wodurch Membranen mit Phenyl-Liganden (Ph-Membranen) erhalten wurden.

**Beispiel 10**

Einführung von Butyl-Liganden (Butyl-Membran)

Aktivierend vernetzte Membranen (Zwischenprodukte) wurden drei Stunden bei Raumtemperatur mit einer wässrigen Lösung von 1 % Butylamin in 0,1 M KPi-Puffer bei einem pH-Wert von 11,4 behandelt und die feuchten Proben wurden 19 h im verschlossenen Gefäß belassen. Nach 15 min Spülen mit fließendem Wasser wurde 15 Minuten mit 1 M wässriger NaCl-Lösung und 15 min mit fließendem Wasser nachgespült, wodurch Membranen mit Butyl-Liganden (Butyl-Membranen) erhalten wurden.

**Beispiel 11**

Einführung von Hexyl-Liganden (Hexyl-Membran)

Aktivierend vernetzte Membranen (Zwischenprodukte) wurden drei Stunden bei Raumtemperatur mit einer wässrigen Lösung von 1 % Hexylamin in 0,1 M KPi-Puffer bei einem pH-Wert von 11,3 behandelt und die feuchten Proben wurden 19 h im verschlossenen Gefäß belassen. Nach 15 min Spülen mit fließendem Wasser wurde 15 min mit 1 M wässriger NaCl-Lösung und 15 min mit fließendem Wasser nachgespült, wodurch Membranen mit Hexyl-Liganden (Hexyl-Membranen) erhalten wurden.

**Beispiel 12**

Einführung von Octyl-Liganden (Octyl-Membran)

Aktivierend vernetzte Membranen (Zwischenprodukte) wurden 3 Stunden bei Raumtemperatur mit einer Lösung von 1 % Octylamin und 35 % Diglyme in 0,1 M KPi-Puffer bei einem pH-Wert von 10,9 behandelt und die feuchten Proben wurden 19 h im verschlossenen Gefäß belassen. Nach 15 min Spülen mit fließendem Wasser wurde 15 min mit 1 M wässriger NaCl-Lösung und 15 min mit fließendem Wasser nachgespült, wodurch Membranen mit Octyl-Liganden (Octyl-Membranen) erhalten wurden.

**Beispiel 13**

Trocknung der erfindungsgemäßen Adsorptionsmembran

CA-Membranen wurden in 0,5 M wässriger Natronlauge-Lösung 30 Minuten bei Raumtemperatur verseift, anschließend ohne Spülung mit einer Lösung von 30%igem 1,4-Butandioldiglycidylether und 0,1%igem Natriumborhydrid in 0,5 M wässriger Natronlauge-Lösung bei Raumtemperatur 2,5 Stunden vernetzt, danach mit Trimethylamin derivatisiert und sowohl im ungetrockneten Zustand als auch im bei 80 °C im Umlufttrockenschrank getrockneten Zustand hinsichtlich ihrer statischen Bindungskapazität untersucht. Die Ergebnisse sind in der Tabelle 5 angegeben.

**Beispiel 14**

Bestimmung des Anteils an Ultraporen am Gesamtporenvolumen der Membranen

Für die Bestimmung des Anteils an Ultraporen am Gesamtvolumen der Membranen wurde die CA-Membran bei unterschiedlichen Natronlaugekonzentrationen analog zu Beispiel 2 verseift, mit 0,5M NaOH gespült, wie im Beispiel 2 vernetzt und mit Sulfonsäureliganden wie im Beispiel 8 modifiziert (Membranen 1-6). Zum Vergleich wurden die Membran aus Beispiel 1 (Membran A) und die im Stand der Technik bekannten Mikrofiltrationsmembranen der Fa. Sartorius Stedim Biotech GmbH mit Porengrößen im Bereich 0,2-0,45 $\mu$m (Membranen B-F) verwendet.
Als Dextranblau wurde im Handel erhältliches Dextran aus Leuconostoc mesenteroides, Strain B 512, modifiziert mit Reactive Blue 2 dye, ca. 0,1 mmol Reactive Blue 2 pro Gramm Dextran (Blue Dextran Molecular Weight (Mw) 2.000.000 von Sigma, St. Louis, Missouri, USA, Produktnummer D 5751, CAS-Nummer: 87915-38-6), verwendet.
Der hydrodynamische Durchmesser d dieses Dextranblaus kann mit Hilfe der Gleichung nach Mark-Houwink-Sakurada:

$$d[nm]=0,054 \times Mw^{0,5}$$

berechnet werden und beträgt 76,4 nm.
Ultraporen sind, wie vorstehend definiert, Poren, die für Dextranblau nicht zugänglich sind.
Das für Wasser zugängliche Porenvolumen wird als Vw [cm$^3$] bezeichnet. Es wird angenommen, daß alle Membranporen für Wasser zugänglich sind, so daß Vw dem Gesamtporenvolumen der Membran entspricht.
Das für Dextranblau zugängliche Porenvolumen wird als Vd [cm$^3$] bezeichnet.
Das für Dextranblau nicht zugängliche Porenvolumen der Ultraporen wird als Vp [cm$^3$] bezeichnet.
Vp wird durch das erfindungsgemäße Verfahren, bei dem die Celluloseester-Membran während der Verseifung quillt, vergrößert.

$$\text{Es gilt Vw = Vd + Vp und Vp = Vw - Vd}$$

Der prozentuale Anteil von für Dextranblau unzugänglichen Poren in der Membran ist

$$\% \; Vp = 100 \; x \; (Vw - Vd)/Vw.$$

Das für Dextranblau zugängliche Porenvolumen Vd wird nach folgendem Verfahren bestimmt:

10 ml einer Lösung von Dextranblau in RO-Wasser bekannter Konzentration (c0) wird durch eine nasse Membran filtriert. Dadurch wird das Wasser aus dem für Dextranblau zugänglichen Porenvolumen mit der Dextranblau-Lösung ausgetauscht. Die Voraussetzung für die Methode ist, daß die Membran das Dextranblau nicht adsorptiv bindet. Dies ist für unmodifizierte Cellulosehydrat-Membranen, vernetzt und unvernetzt, gegeben. Eine Membran mit einem Durchmesser von 50 mm (d.h. einer Fläche von 19,6 cm$^2$) wird mit fließendem RO-Wasser 15 Minuten intensiv gewaschen. Die nasse Membran wird dann in ein Filtrationsgehäuse eingebaut und 10 ml Dextranblau-Lösung mit einer Konzentration von 5 mg/ml (c0) werden bei 0,1 bar Druck durch die Membran filtriert. Dann wird die Membran aus dem Filtrationsgehäuse ausgebaut, ein Stanzling mit einem Durchmesser von 47 mm (d.h. einer Fläche von 17,3 cm$^2$) wird mittig ausgestanzt (um die abgedichteten Ränder der Membran zu entfernen) und mit einem Labortuch (Kimtech Science, 200, 2, 21 x 20 cm, weiß, 7101) abgetupft.

Danach wird die Membran in einer genau bestimmten Menge (Volumen V = 5,0 ml) RO-Wasser in einem abgedichteten Gefäß 20 Stunden bei 80 Upm geschüttelt. Die Konzentration der Dextranblau-Lösung (c) wird dann photometrisch bei 618 nm bestimmt. Der Extinktionskoeffizient E (1 mg/ml; 1 cm) der Dextranblau-Lösung beträgt 0,896. Aus der Konzentration der Dextranblau-Lösung wird das für Dextranblau zugängliche Porenvolumen berechnet:

$$Vd \; [cm^3] = c \; x \; V/c0$$

Das für Wasser zugängliche Porenvolumen wird nach folgendem Verfahren bestimmt:

Die Membranprobe wird mit fließendem RO-Wasser 15 Minuten intensiv gewaschen. Das an der Membran anhaftende Wasser wird mit dem Labortuch abgetupft und die nasse Membran wird gewogen. Danach wird die Membran bei 80 °C in einem Umlufttrockenschrank 30 Minuten getrocknet und die getrocknete Membran wird gewogen. Die Gewichtsdifferenz zwischen nasser und trockener Membran entspricht der Wassermenge in der Membran (Vw). Dabei wird eine Wasserdichte von 1,0 g/cm$^3$ angenommen.

$$Aus \; \% \; Vp = 100 \; x \; (Vw - Vd)/Vw$$

wird der prozentuale Anteil des für Dextranblau nicht zugänglichen Porenvolumens am Gesamtporenvolumen berechnet.

Mit steigender Natronlaugekonzentration bei der Verseifung der Celluloseacetat-Membran wird die Quellung stärker, der Quellungsgrad steigt, die Permeabilität der Membran sinkt, die Membrandicke nimmt zu, der Anteil an Ultraporen am Gesamtporenvolumen nimmt zu und die Bindungskapazität steigt, wie aus der folgenden Tabelle 1 ersichtlich ist.

Tabelle 1

|  | Verseifung | Spülung nach Verseifung | Vp | Permeabilität 10mM Kpi pH7 | Bindungskapazität Lysozym | Quellungsgrad |
|---|---|---|---|---|---|---|
|  | c (NaOH) [M] | c(NaOH) [M] | [%] | S Membran [ml/(min x bar x cm$^2$)] | S Membran [mg/cm$^2$] | [-] |
| 1 | 0,20 | 0,5 | 16% | 515 | 0,75 | 1,4 |
| 2 | 0,40 | 0,5 | 25% | 341 | 1,40 | 2,1 |
| 3 | 0,50 | 0,5 | 30% | 180 | 1,71 | 4,1 |
| 4 | 0,60 | 0,5 | 34% | 73 | 1,99 | 10,0 |
| 5 | 0,75 | 0,5 | 39% | 8 | 2,23 | 90,1 |

(fortgesetzt)

| | Verseifung | Spülung nach Verseifung | Vp | Permeabilität 10mM Kpi pH7 | Bindungskapazität Lysozym | Quellungsgrad |
|---|---|---|---|---|---|---|
| | c (NaOH) [M] | c(NaOH) [M] | [%] | S Membran [ml/(min x bar x cm$^2$)] | S Membran [mg/cm$^2$] | [-] |
| 6 | 1,00 | 0,5 | 45% | 4 | 2,40 | 208,6 |

**Beispiel 15 Proteintrennung**

Jeweils 5 Lagen von Membranen der Beispiele 9, 10, 11 und 12 wurden übereinander angeordnet und der Membranstapel wurde in einen Membranhalter eingespannt. Jeder Membranstapel mit einer jeweiligen aktiven Membranfläche von 100 cm$^2$, einer Anströmfläche von 20 cm$^2$ und einer Bedhöhe (Dicke des Membranstapels) von 1,4 mm in dem Membranhalter wurde mit RO-Wasser geflutet, um die Luft aus den Membranen zu verdrängen und anschließend an eine Fast Protein Liquid Chromatography (FPLC)-Anlage (Äkta Prime der Firma General Electric Health Care) angeschlossen. Danach wurden die Membranen bzw. Membranstapel mit einem vier Schritte umfassenden Testprogramm hinsichtlich der Proteintrennung untersucht. In dem Testprogramm werden als Puffer A eine 1,7 M (NH$_4$)$_2$SO$_4$-Lösung in 50 mM KPi-Puffer mit einem pH-Wert von 7,0 und als Puffer B ein 50 mM KPi-Puffer mit einem pH-Wert von 7,0 verwendet. Die vier Schritte des Testprogramms sind nachfolgend angegeben:

1. Äqulibrieren der Membranen mit 20 ml des Puffers A, die mit einer Flußgeschwindigkeit von 10 ml/min durch die Membranen strömen;
2. Beladen der Membranen mit 2 ml einer mittels eines 0,45 µm Filters vorfiltrierten Lösung von 0,5 mg/ml Cytochrom C, 0,5 mg/ml a-Chymotrypsinogen, 0,5 mg/ml Lysozym und 0,5 mg/ml Myoglobin in Puffer A, die über einen sog. Loop mit einer Flußgeschwindigkeit von 10 ml/min aufgetragen wird,
3. Waschen der beladenen Membran mit 18 ml des Puffers A mit einer Flußgeschwindigkeit von 10 ml/min und
4. Eluieren der beladenen und gewaschenen Membranen mit 150 ml des Puffers B in Puffer A mit einem linearen Gradienten von 40-100 % mit einer Flußgeschwindigkeit von 10 ml/min.

Das Eluat wurde durch Messung der optischen Dichte (OD) bei 280 nm hinsichtlich der Menge der eluierten Proteine und deren Trennung untersucht. Das Ergebnis ist in der Tabelle 2 angegeben und in den Figuren 6, 7, 8 und 9 graphisch dargestellt.

Tabelle 2: Proteintrennung nach Beispiel 15

| Membran | Peakposition | Peak 1 | Peak 2 | Peak 3 | Peak 4 |
|---|---|---|---|---|---|
| Beispiel 9 Phenyl-Membran | Retentionsvolumen [ml] | 5,6 | 29,4 | 41,0 | 64,5 |
| | Leitfähigkeit [mS/cm] | 177 | 160 | 121 | 105 |
| | Puffer B[%] | 0 | 43,8 | 48,3 | 57,8 |
| Beispiel 10 Butyl-Membran | Retentionsvolumen [ml] | 6,2 | 31,5 | - | - |
| | Leitfähigkeit [mS/cm] | 177 | 141 | - | - |
| | Puffer B[%] | 0 | 44,6 | - | - |
| Beispiel 11 Hexyl-Membran | Retentionsvolumen [ml] | 5,7 | 34,0 | - | - |
| | Leitfähigkeit [mS/cm] | 176 | 131 | - | - |
| | Puffer B[%] | 0 | 45,6 | - | - |
| Beispiel 12 Octyl-Membran | Retentionsvolumen [ml] | 5,5 | 35,6 | 68,3 | - |
| | Leitfähigkeit [mS/cm] | 178 | 128 | 102 | - |
| | Puffer B[%] | 0 | 46,3 | 59,3 | - |

**Auswertung der Membranen**

Die Auswertung der erhaltenen Membranen erfolgte in der nachstehend beschriebenen Weise:

1) Durchflussbestimmung

Membranen mit einer aktiven Membranfläche von 12,5 cm$^2$ wurden jeweils in ein Gehäuse eingebaut und es wurde die Zeit für die Filtration von 100 ml Wasser oder Puffer gemessen. Die in der Tabelle 5 wiedergegebenen Durchflussangaben für mit funktionellen Gruppen umgesetzten Membranen beziehen sich auf den entsprechenden Bindungspuffer. Es wurden die gleichen Puffer verwendet wie für die unten beschriebenen Bestimmungen der Bindungskapazitäten.

2) Bestimmung der statischen Bindungskapazität von Q-Membranen

Membranproben mit einer aktiven Membranfläche von jeweils 17,6 cm$^2$ wurden in 35 ml 20 mM TRIS/HCl pH 7,3 3 x 5 min mit etwa 80 Umdrehungen pro Minute (Upm) geschüttelt. Danach wurden 35 ml einer Lösung von 2 mg/ml Rinderserumalbumin (RSA)-Lösung in 20 mM TRIS/HCl pH 7,3 12-18 Stunden bei 20-25 °C mit etwa 80 Upm geschüttelt. Anschließend wurden die Membranproben 2 x 15 Minuten in jeweils 35 ml 20 mM TRIS/HCl pH 7,3 gespült. Danach wurden die Membranproben in 20 ml 20 mM TRIS/HCl pH 7,3 + 1 M wässriger NaCl-Lösung geschüttelt. Die Menge des eluierten Proteins wurde durch Messung der optischen Dichte (OD) bei 280 nm bestimmt.

3) Bestimmung der statischen Bindungskapazität von S-Membranen

Membranproben mit einer aktiven Membranfläche von jeweils 17,6 cm$^2$ wurden in 35 ml 10 mM KPi pH 7,0 3 mal 5 Minuten mit etwa 80 Upm geschüttelt. Danach wurden 35 ml einer Lösung von 2 mg/ml Lysozym in 10 mM KPi pH 7,6 12-18 Stunden bei 20 - 25 °C mit etwa 80 Upm geschüttelt. Anschließend wurden die Membranproben 2 x 15 Minuten in jeweils 35 ml 10 mM KPi pH 7,0 gespült. Danach wurden die Membranproben in 20 ml 10 mM KPi pH 7,0 + 1 M wässriger NaCl-Lösung geschüttelt. Die Menge des eluierten Proteins wurde durch Messung der optischen Dichte (OD) bei 280 nm bestimmt.

4) Bestimmung der statischen Bindungskapazität von Ph-Membranen, Butyl-Membranen, Hexyl-Membranen und Octyl-Membranen

Membranproben mit einer aktiven Membranfläche von 3,1 cm$^2$ wurden in einen Polycarbonatvorsatz eingespannt und an eine Schlauchpumpe angeschlossen. Lösungen in folgender Reihenfolge wurden mit einer Flußgeschwindigkeit von 2 ml/min mit Hilfe der Schlauchpumpe durch die Membranen gepumpt:

1. 10 ml 0,05 M KPi + 1 M $(NH_4)_2 SO_4$ pH 7,0
2. 20 ml 1 mg/ml gamma-Globulin in 0,05 M KPi + 1 M $(NH_4)_2SO_4$ pH 7,0
3. 20 ml 0,05 M KPi + 1M $(NH_4)_2 SO_4$ pH 7,0
4. 10 ml 0,05 M KPi pH 7,0

Die Menge des eluierten Proteins in Schritt 4 wurde durch Messung der optischen Dichte (OD) bei 280 nm bestimmt.

5) Bestimmung der Laufzeit

Die Membranproben aus den Beispielen 2, 9, 10, 11 und 12 wurden 20 Minuten bei 80 °C in einem Umlufttrockenschrank getrocknet. Nach einer fünfminütigen Abkühlung auf Raumtemperatur wurden jeweils 10 μl einer 5,8%igen wässrigen NaCl-Lösung auf die jeweilige Membran aufgebracht und die Zeit bis zum vollständigen Aufsaugen der Lösung in die Membran mit einer Stoppuhr gemessen. Die Laufzeit ist in Sekunden angegeben.

Das Ergebnis der Bestimmung des Durchflusses und der Laufzeit der Membranen aus den Beispielen 2, 9, 10, 11 und 12 ist in der nachfolgenden Tabelle 3 angegeben.

Tabelle 3

| Beispiel | Ligand | Durchfluss | Bindungskapazität | Laufzeit |
|---|---|---|---|---|
| 2 | keiner (Basismembran) | 84 | 0,03 | 5 |

(fortgesetzt)

| Beispiel | Ligand | Durchfluss | Bindungskapazität | Laufzeit |
|---|---|---|---|---|
| 9 | Phenyl | 90 | 0,82 | 10 |
| 10 | Butyl | 88 | 0,25 | 7 |
| 11 | Hexyl | 84 | 0,67 | 36 |
| 12 | Octyl | 112 | 0,66 | 617 |

6) Bestimmung der Bindungskapazität in Abhängigkeit von der Konzentration des Ammoniumsulfats im Puffer

Die Bindungskapazitäten einer wie im Beispiel 9 hergestellten Phenyl-Membran wurden wie in Punkt 4) "Bestimmung der statischen Bindungskapazität von Ph-Membranen, Butyl-Membranen, Hexyl-Membranen und Octyl-Membranen" in Abschnitt "Auswertung der Membranen" beschrieben, bestimmt, mit dem Unterschied, daß die Konzentration von Ammoniumsulfat von 0 M bis 1,0 M variiert wurde und die Membranen nach jedem Zyklus der Schritte 1 bis 4 in einem zusätzlichen Schritt 5 mit 10 ml einer 50%igen Lösung von Ethylenglycol in RO-Wasser regeneriert wurden. Die erhaltenen Meßwerte sind in Tabelle 4 angegeben und in Figur 10 graphisch dargestellt.

Tabelle 4

| Ammoniumsulfat | Bindungskapazität Basismembran | Bindungskapazität Phenyl-Membran |
|---|---|---|
| [M] | Beispiel 2 | Beispiel 9 |
| 0 | 0,008 | 0,002 |
| 0,2 | 0,025 | 0,025 |
| 0,4 | 0,020 | 0,081 |
| 0,6 | 0,031 | 0,210 |
| 0,8 | 0,052 | 0,456 |
| 1,0 | 0,078 | 0,686 |

7) Konfokale Laserfluoreszenzmikroskopie

Markierung der Membranen

Eine Adsorptionsmembran aus Beispiel 1 und eine Adsorptionsmembran nach Beispiel 2 wurden nach Beispiel 8 mit Sulfonsäure-liganden versehen. Zusammen mit einer unter der Bezeichnung Sartobind® S im Handel erhältlichen Adsorptionsmembran der Sartorius Stedim Biotech GmbH mit einem mit Sulfonsäure-belegten Hilfspolymer wurden die Membranen mit dem OH-reaktiven Fluoreszenzfarbstoff "5-DTAF" (5-(4,6-Dichlorotriazinyl)aminofluorescein, Anregungs- bzw. Emissionswellenlänge von 492 nm bzw. 516 nm, Firma Invitrogen) markiert. Die Inkubation der Lösung des Farbstoffs sowie alle folgenden Waschschritte wurden mit jeweils drei Membranproben (Durchmesser: 13 mm) in einem Filterhalter bei kontinuierlicher Durchspülung mit einem Durchfluß von ca. 1 ml/min durchgeführt. Dabei wurden jeweils 20 ml einer 5-DTAF-Lösung in 100 mM Natriumhydrogencarbonat-Lösung mit einem pH-Wert von 9,3 mit an die Membran angepaßten Konzentrationen, nämlich 13,5 $\mu$g/ml 5-DTAF + 100 mM NaCl-Lösung für die Membranen nach den Beispielen 1 und 2 und 25 $\mu$g/ml 5-DTAF + 200 mM NaCl-Lösung für die Sartobind® S Membran, verwendet. Da vermutet wurde, daß die dreidimensionale Kationenaustauscherschicht eine besonders effektive Abschirmung der Cellulosematrix bewirkt, wurde für die Sartobind® S Membran sowohl die Farbstoffals auch die Salzkonzentration erhöht. Nach Durchspülung für ca. 18 Stunden wurden die Proben anschließend nacheinander mit jeweils 100 ml einer 20%igen Ethanollösung, 1 M NaCl-Lösung und 200 mM Natriumphosphat-Puffer, pH 7,0, gewaschen. Für die CLSM-Analyse wurde wegen der besten Homogenität der Markierung jeweils die zweite Probe im Filterhalter benutzt.

Markierung und Reinigung des Proteins

Lysozym (erhältlich von der Firma Sigma, St. Louis, Missouri, USA; Protein ca. 95%, ca. 50 000 units/mg Protein) wurde mit dem $NH_2$-reaktiven Fluoreszenzfarbstoff "Cy5 mono-reactive NHS Ester" (erhältlich von der Firma GE Health Care Bio-Sciences AB, Uppsala, Schweden) in Natriumcarbonat-Puffer, pH 9,3, markiert sowie anschließend zunächst durch Gelfiltration und dann durch HPIonenaustauschchromatographie gereinigt. Durch entsprechende Auswahl der Fraktionen der Chromatographie wurde das einfach markierte Lysozym in reiner Form erhalten. Danach erfolgte mittels Ultrafiltration die Aufkonzentrierung auf die für das Bindungsexperiment notwendige Konzentration. Die Bestimmung der Konzentration des markierten Lysozyms erfolgte mittels UV-Vis-Photometer (Messung der Absorptionen bei 280 nm und 650 nm).

Inkubation der Membranen mit Protein

Proben der mit 5-DTAF-markierten Membranen wurden mit einem Durchmesser von 5 mm ausgestanzt und für vier Stunden in einer Lösung des markierten Lysozyms mit einer Konzentration von 0,6 g/L in 200 mM Natriumphosphat-Puffer, pH 7,0 + 50 mM wässrige NaCl-Lösung inkubiert (für 1 cm$^2$ Proben wurden jeweils 4,1 ml Proteinlösung eingesetzt). Danach wurden die Proben mit dem Puffer für 15 Minuten gewaschen.

CLSM Analyse

Die Analyse erfolgte mit dem CLSM-System Leica TCS SP. Jede Probe wurde in 200 mM Natriumphosphat-Puffer von beiden Oberflächen aus untersucht. Zunächst erfolgte die Bestimmung der geeigneten Signalverstärkung (Kriterien: unterdrückte Autofluoreszenz der Membran; das Maximum der Signalverstärkung wurde mit Hilfe des Histogramms in der Auswertesoftware "Zeiss LSM Image Browser" eingestellt, um örtliche Überbelichtungen zu vermeiden) und Identifizierung von z = 0 (Kriterien: hohe Streuintensität und anschließende erstmalige Identifizierung der Porenmorphologie bei weiterer Verringerung des Abstands zur Probe). Danach wurde in x,y-Scans an verschiedenen z-Positionen die aus SEM bekannte charakteristische Morphologie der Sartobind® S Membranen gesucht. Danach erfolgten in einem engen Bereich verschiedener z-Positionen (in ca. 20 Mikrometer Tiefe, in Abständen von 1 Mikrometer in beide Richtungen) detaillierte x, y-Scans der beiden Anregungswellenlängen (488 nm für 5-DTAF, 633 nm für Cy5). Für jede Probe und jede Orientierung wurden diese Scans jeweils für drei verschiedene Positionen durchgeführt. Die Probe Sartobind® S wurde zuerst vermessen; die für diese Probe gewählten Einstellungen (z-Position und Signalverstärkung) wurden bei der Messung der anderen Membranproben beibehalten. Weil die Signalintensitäten von der Membran nach Beispiel 2 bei 633 nm sehr viel höher als bei den anderen beiden Membranen waren, wurde eine Verringerung der Signalverstärkung vorgenommen:

"Gains" (488 nm/633nm)

Membran Sartobind® S: 426/643

Membran nach Beispiel 1: 426/669

Membran nach Beispiel 2: 357/650

CLSM Auswertung

Die Auswertungen erfolgten mit Hilfe des Zeiss LSM Image Browsers 3.5.0.376. Aus den erhaltenen Bildern wurden detaillierte x,y-Scans in einem Bereich der z-Positionen von ca. 20 μm Tiefe für die Oberseite ausgewählt. Die erhaltenen Bilder wurden jeweils als 8-Bit Bilder mit einer Auflösung von 512 x 512 Pixel, entsprechend 146,2 x 146,2 μm$^2$, dargestellt. Die Figuren 2 bis 4 zeigen die Überlappung der beiden Bilder der Verteilung von Lysozym und der Porenmorphologie der Cellulose. Zusätzlich wird für jede Messung am oberen und rechten Rand der Darstellung auch noch ein Intensitätsprofil der Intensitäten für beide Fluoreszenzmarkierungen gezeigt.

Ergebnisse der Versuche

Die Ergebnisse der Versuche sind in der nachstehenden Tabelle 5 gezeigt.

Tabelle 5

| Membran aus Beispiel | Bemerkung | Ligand | Protein | Durchfluss | Bindungskapazität |
|---|---|---|---|---|---|
| 1 | | Q | RSA | 643 | 0,07 |
| | | S | Lysozym | 664 | 0,01 |
| | | Ph | gamma-Globulin | 570 | 0,2 |

(fortgesetzt)

| Membran aus Beispiel | Bemerkung | Ligand | Protein | Durchfluss | Bindungskapazität |
|---|---|---|---|---|---|
| 2 | | Q | RSA | 44 | 0,92 |
| | | S | Lysozym | 38 | 2,06 |
| | | Ph | gamma-Globulin | 31 | 1,26 |
| 3 | | Q | RSA | 20 | 1,13 |
| | | S | Lysozym | 24 | 2,85 |
| 4 | | Q | RSA | 158 | 0,74 |
| | | S | Lysozym | 167 | 3,11 |
| | | S | gamma-Globulin | 167 | 0,44 |
| 5 | LiOH | Q | RSA | 70 | 1,18 |
| | NaOH | Q | RSA | 109 | 0,93 |
| | KOH | Q | RSA | 519 | 0,08 |
| 13 | ungetrocknet getrocknet | Q | RSA | 213 | 0,94 |
| | | Q | RSA | 239 | 0,92 |

**Vergleichsbeispiel 1**

Gleichzeitige Verseifung und Vernetzung wie im Beispiel 1, Probe K10C der WO 2007/017085 A2, aber mit 1,4-Butandioldiglycidylether anstelle von Epichlorhydrin, unter nicht-quellenden Bedingungen.

Es wurden eine wie vorstehend definierte CA-Membran und eine 0,65 $\mu$m Celluloseacetat-Membran wie in Bsp. 1, Probe K10C der WO 2007/017085 A2 mit einem Wasserdurchfluß von 65 ml/(min x bar x cm$^2$) als Ausgangs-membranen verwendet.

Die Celluloseacetatmembranen wurden in 100 g Wasser, 10 g Na$_2$SO$_4$ und 1 g 1,4-Butandioldiglycidylether auf 47 °C erhitzt und 10 g einer 1 M wässrigen Natronlauge-Lösung wurden während 30 Minuten zudosiert. Die Membranen wurden weiter in der Lösung 3,5 Stunden bei 47 °C behandelt und anschließend 30 Minuten mit fließendem Wasser gespült. In die Membranen wurden quaternäre Ammonium-Liganden eingeführt, wobei Q-Membranen erhalten wurden. Die aus der CA-Membran erhaltene, verseifte und vernetzte Q-Membran wies einen Wasserdurchfluss von 589 ml/(min x bar x cm$^2$), einen Quellungsgrad von 1,2 und eine Bindungskapazität für RSA von 0,04 mg/cm$^2$ auf. Die aus der Celluloseacetat-Membran gemäß Beispiel 1, Probe K10C der WO 2007/017085 A2 erhaltene, verseifte und vernetzte Q-Membran wies einen Wasserdurchfluss von 66 ml/(min x bar x cm$^2$), einen Quellungsgrad von 1,0 und eine Bindungskapazität für RSA von 0,04 mg/cm$^2$ auf.

**Vergleichsbeispiel 2** Versuch zur Quellung von bereits verseiften Cellulosehydrat-Membranen

Eine als Ausgangsmembran verwendete und wie vorstehend definierte CA-Membran wurde drei Minuten bei Raumtemperatur mit einer 15%igen Kalilauge-Lösung in 80%igem Ethanol verseift und anschließend drei Minuten mit einer 6,8%igen Essigsäure-Lösung, zweimal mit Ethanol und 15 Minuten mit fließendem RO-Wasser gespült. Die erhaltene, verseifte Membran wurde 30 Minuten bei Raumtemperatur mit einer 0,6 M wässrigen Natronlauge behandelt und danach dreimal 10 Minuten mit einer 0,5 M wässrigen Natronlauge gespült. Anschließend wurde die Membran 30 Minuten bei Raumtemperatur mit einer 30%igen Lösung von 1,4-Butandioldiglycidylether in 0,1 M wässriger Natronlauge-Lösung und 0,1%iger, wässriger Natriumborhydrid-Lösung behandelt, worauf die feuchte Membran 20 Stunden in einem abgeschlossenen Gefäß bei Raumtemperatur stehengelassen wurde. Schließlich wurde die erhaltene Membran 30 Minuten mit fließendem Wasser gespült.

Der Wasserdurchfluss der so hergestellten, verseiften und vernetzten Cellulosehydrat-Membran betrug 688 ml/(min x bar x cm$^2$) und der Quellungsgrad betrug 1,06.

In zwei Proben der Membran wurden, wie in den Beispielen 7 bzw. 8 beschrieben, quaternäre Ammoniumliganden bzw. Sulfonsäureliganden eingeführt, wodurch eine Q-Membran und eine S-Membran erhalten wurden. Die Q-Membran wies eine Bindungskapazität für RSA von 0,044 mg/cm$^2$ auf und die S-Membran eine Bindungskapazität für Lysozym von 0,067 mg/cm$^2$.

**Patentansprüche**

1. Verfahren zur Herstellung einer vernetzten Cellulosehydrat-Membran mit einer porösen Doppelstruktur, welche besteht aus

   - Mikroporen mit einem Durchmesser im Bereich von >100 nm bis 20 µm, und
   - Ultraporen mit einem Durchmesser im Bereich von <100 nm, die für Dextranblau mit einem mittleren Molekulargewicht Mw von 2.000.000 nicht zugänglich sind,

   wobei der Anteil des Volumens der Ultraporen an dem für Wasser zugänglichen Gesamtporenvolumen mehr als 15 % beträgt und
   wobei hydrophobe Liganden, ausgewählt aus
   C1-C20-Alkyl und deren Derivaten oder C6-C25-Aryl und deren Derivaten oder C7-C25-Arylalkyl und deren Derivaten oder -[(CH2)m-O-]n-R, wobei m 2 oder 3 ist, n eine ganze Zahl größer gleich 1 ist und R -H oder -C1-C5-Alkyl bedeuten, an die Membran gebunden sind, umfassend die Schritte:

   - Bereitstellen einer Celluloseester-Membran mit einem Porendurchmesser von 0,1 bis 20 µm;
   - gegebenenfalls Vorbehandeln der Celluloseester-Membran in einem Medium bei einer Temperatur von 20°C bis 100°C;
   - Verseifen der gegebenenfalls vorbehandelten Celluloseester-Membran unter quellenden Bedingungen in einem Natriumhydroxid oder Lithiumhydroxid enthaltenden wässrigen Verseifungsmedium, wobei die Konzentration des Natriumhydroxids oder Lithiumhydroxids im Verseifungsmedium 0,4 Gew.-% bis 50 Gew.-%, bezogen auf das Verseifungsmedium, beträgt;
   - nachfolgendes Vernetzen der verseiften Membran mit mindestens einem Vernetzungsmittel, welches bzw. welche mindestens 2 funktionelle Gruppen im Molekül aufweist bzw. aufweisen, die mit den Hydroxylgruppen der Cellulose reaktiv sind; und
   - Einführen von hydrophoben Liganden in die Membran, wobei die hydrophoben Liganden aus C1-C20-Alkyl und deren Derivaten oder C6-C25-Aryl und deren Derivaten oder C7-C25-Arylalkyl und deren Derivaten oder -[(CH2)m-O-]n-R, wobei m 2 oder 3 ist, n eine ganze Zahl größer gleich 1 ist und R -H oder -C1-C5-Alkyl bedeuten, ausgewählt sind.

2. Verfahren nach Anspruch 1, wobei das Verseifungsmedium mindestens ein quellungsbeeinflussendes Additiv enthält.

3. Verfahren nach Anspruch 1 oder 2, wobei die Temperatur des Verseifungsmediums während der Verseifung von 10°C bis zum Siedepunkt des Verseifungsmediums beträgt.

4. Verfahren nach einem oder mehreren der Ansprüche 1 bis 3, wobei die Verseifung in einem Zeitraum von 0,1 bis 60 min durchgeführt wird.

5. Verfahren nach einem oder mehreren der Ansprüche 1 bis 4, wobei das Vernetzungsmittel ausgewählt ist aus der Gruppe von Diepoxidverbindungen, Diisocyanaten, Epichlorhydrin, Epibromhydrin, Dimethylharnstoff, Dimethylethylenharnstoff, Dimethylchlorsilan, Bis-(2-Hydroxyethylsulfon), Divinylsulfon, Alkylen-Dihalogen, Hydroxyalkylen-Dihalogen und Glycidylethern.

6. Verfahren nach einem oder mehreren der Ansprüche 1 bis 5, wobei die Vernetzung in einem wässrigen Medium, einem organischen Lösungsmittel oder einem Gemisch aus Wasser und einem organischen Lösungsmittel durchgeführt wird.

7. Verfahren nach einem oder mehreren der Ansprüche 1 bis 6, wobei die Vernetzung in Anwesenheit eines Vernetzungskatalysators durchgeführt wird.

8. Verfahren nach einem oder mehreren der Ansprüche 1 bis 7, wobei die Vernetzung bei einer Temperatur von etwa 4°C bis zum Siedepunkt des Vernetzungsmediums durchgeführt wird.

9. Verfahren nach einem oder mehreren der Ansprüche 1 bis 8, wobei die Vernetzung in einem Zeitraum von 10 Minuten bis 100 Stunden durchgeführt wird.

**10.** Verfahren nach einem oder mehreren der Ansprüche 1 bis 9, wobei die hydrophoben Liganden während der Vernetzung oder nach der Vernetzung eingeführt werden.

**11.** Verfahren nach Anspruch 10, wobei die Membran nach Einführung der hydrophoben Liganden getrocknet wird.

**12.** Verfahren nach Anspruch 11, wobei die Membran aus einem Medium getrocknet wird, das eine porenstabilisierende Verbindung enthält.

**13.** Vernetzte Cellulosehydrat-Membran mit einer porösen Doppelstruktur, hergestellt nach dem Verfahren des Anspruchs 1, welche besteht aus

- Mikroporen mit einem Durchmesser im Bereich von >100 nm bis 20 $\mu$m, und
- Ultraporen mit einem Durchmesser im Bereich von <100 nm, die für Dextranblau mit einem mittleren Molekulargewicht Mw von 2.000.000 nicht zugänglich sind,

wobei der Anteil des Volumens der Ultraporen an dem für Wasser zugänglichen Gesamtporenvolumen mehr als 15 % beträgt und
wobei hydrophobe Liganden, ausgewählt aus
C1-C20-Alkyl und deren Derivaten oder C6-C25-Aryl und deren Derivaten oder C7-C25-Arylalkyl und deren Derivaten oder -[(CH2)m-O-]n-R, wobei m 2 oder 3 ist, n eine ganze Zahl größer gleich 1 ist und R -H oder -C1-C5-Alkyl bedeuten, an die Membran gebunden sind.

**14.** Vorrichtung zur hydrophoben Interaktionschromatographie, umfassend mindestens eine vernetzte Cellulosehydrat-Membran nach Anspruch 13.

**15.** Verwendung der vernetzten Cellulosehydrat-Membran nach Anspruch 13 in der hydrophoben Interaktionschromatographie.

**16.** Verwendung der vernetzten Cellulosehydrat-Membran nach Anspruch 13 zur Kontaminantenentfernung.

**17.** Verwendung der vernetzten Cellulosehydrat-Membran nach Anspruch 13 für selektive Adsorption und anschließende Desorption einer oder mehrerer Zielsubstanzen.

**Claims**

**1.** A method for producing a crosslinked cellulose hydrate membrane having a porous double structure, consisting of

- micropores having a diameter in the range of >100 nm to 20 $\mu$m, and
- ultrapores having a diameter in the range of <100 nm, which are not accessible to Blue Dextran having an average molecular weight Mw of 2 000 000,

wherein the fraction of the volume of the ultrapores is more than 15% of the entire pore volume accessible to water, and
wherein hydrophobic ligands, selected from
C1-C20-alkyl and their derivatives or C6-C25-aryl and their derivatives or C7-C25-arylalkyl and their derivatives or -[(CH2)m-O-]n-R, where m is 2 or 3, n is an integer greater than or equal to 1, and R is -H or -C1-C5-alkyl,
are bonded to the membrane, comprising the steps:

- providing a cellulose ester membrane having a pore diameter of from 0.1 to 20 $\mu$m;
- optionally pretreating the cellulose ester membrane in a medium at a temperature of from 20°C to 100°C;
- hydrolyzing the optionally pretreated cellulose ester membrane under swelling conditions in an aqueous hydrolysis medium containing sodium hydroxide or lithium hydroxide, wherein the concentration of the sodium hydroxide or lithium hydroxide in the hydrolysis medium is from 0.4% by weight to 50% by weight, based on the hydrolysis medium;
- subsequently, crosslinking the hydrolyzed membrane with at least one crosslinking agent which has at least 2 functional groups in the molecule which are reactive with the hydroxyl groups of the cellulose; and
- introducing hydrophobic ligands into the membrane, wherein the hydrophobic ligands are selected from C1-C20-alkyl and their derivatives or C6-C25-aryl and their derivatives or C7-C25-arylalkyl and their derivatives or -[(CH2)m-O-]n-R, where m is 2 or 3, n is an integer greater than or equal to 1, and R is -H or -C1-C5-alkyl.

2. The method according to claim 1, wherein the hydrolysis medium comprises at least one swelling-influencing additive.

3. The method according to claim 1 or 2, wherein the temperature of the hydrolysis medium during the hydrolysis is from 10°C up to the boiling point of the hydrolysis medium.

4. The method according to any one of claims 1 to 3, wherein the hydrolysis is carried out in a period of from 0.1 to 60 minutes.

5. The method according to any one of claims 1 to 4, wherein the crosslinking agent is selected from the group consisting of diepoxide compounds, diisocyanates, epichlorohydrin, epibromohydrin, dimethylurea, dimethylethyleneurea, dimethylchlorosilane, bis(2-hydroxyethyl) sulfone, divinyl sulfone, alkylene dihalogen, hydroxyalkylene dihalogen, and glycidyl ethers.

6. The method according to any one of claims 1 to 5, wherein the crosslinking is carried out in an aqueous medium, an organic solvent, or a mixture of water and an organic solvent.

7. The method according to any one of claims 1 to 6, wherein the crosslinking is carried out in the presence of a crosslinking catalyst.

8. The method according to any one of claims 1 to 7, wherein the crosslinking is carried out at a temperature of from about 4°C up to the boiling point of the crosslinking medium.

9. The method according to any one of claims 1 to 8, wherein the crosslinking is carried out in a period of from 10 minutes to 100 hours.

10. The method according to any one of claims 1 to 9, wherein the hydrophobic ligands are introduced during the crosslinking or after the crosslinking.

11. The method according to claim 10, wherein the membrane is dried after introducing the hydrophobic ligands.

12. The method according to claim 11, wherein the membrane is dried from a medium which comprises a pore-stabilizing compound.

13. A crosslinked cellulose hydrate membrane having a porous double structure produced by the method according to claim 1, consisting of

- micropores having a diameter in the range of >100 nm to 20 $\mu$m, and
- ultrapores having a diameter in the range of <100 nm, which are not accessible to Blue Dextran having an average molecular weight Mw of 2 000 000,

wherein the fraction of the volume of the ultrapores is more than 15% of the entire pore volume accessible to water, and wherein hydrophobic ligands, selected from
C1-C20-alkyl and their derivatives or C6-C25-aryl and their derivatives or C7-C25-arylalkyl and their derivatives or -[(CH2)m-O-]n-R, where m is 2 or 3, n is an integer greater than or equal to 1, and R is -H or -C1-C5-alkyl, are bonded to the membrane.

14. An apparatus for hydrophobic interaction chromatography, comprising at least one crosslinked cellulose hydrate membrane according to claim 13.

15. Use of the crosslinked cellulose hydrate membrane according to claim 13 in hydrophobic interaction chromatography.

16. Use of the crosslinked cellulose hydrate membrane according to claim 13 for contaminant removal.

17. Use of the crosslinked cellulose hydrate membrane according to claim 13 for selective adsorption and subsequent desorption of one or more target substances.

**Revendications**

1.  Procédé de fabrication d'une membrane d'hydrate de cellulose réticulé comportant une structure double poreuse et constituée par

    - des micropores ayant un diamètre dans la gamme allant de >100 nm à 20 $\mu$m, et
    - des ultra-pores ayant un diamètre de <100 nm qui ne sont pas accessibles au bleu de dextrane ayant un poids moléculaire moyen de 2000000,

    la fraction volumétrique des ultra-pores par rapport au volume total de pores accessibles à l'eau étant de plus de 15%, et
    des ligands hydrophobes choisis parmi $C_1$-$C_{20}$-alkyle et dérivés de ceux-ci ou $C_6$-$C_{25}$-aryle et dérivés de ceux-ci ou $C_7$-$C_{25}$-arylalkyle et dérivés de ceux-ci ou -[$(CH_2)_m$-O-]$_n$-R, m étant 2 ou 3, n étant un nombre entier plus grand ou égal à 1 et R étant -H ou -$C_1$-$C_5$-alkyle, sont liés à la membrane, ledit procédé comportant les étapes :

    - mise à disposition d'une membrane d'ester de cellulose ayant un diamètre de pore de 0,1 à 20 $\mu$m ;
    - éventuellement prétraitement de la membrane d'ester de cellulose dans un milieu à une température de 20°C à 100 °C ;
    - saponification de la membrane d'ester de cellulose éventuellement prétraitée dans des conditions de gonflement en milieu aqueux comportant de l'hydroxyde de sodium ou de lithium, la concentration de l'hydroxyde de sodium ou de lithium dans le milieu de saponification étant de 0,4 % en poids à 50 % en poids, par rapport au milieu de saponification ;
    - ensuite réticulation de la membrane saponifiée à l'aide d'au moins un agent de réticulation qui comporte dans la molécule au moins deux groupes fonctionnels qui sont réactifs par rapport aux groupes hydroxyles de la cellulose ; et
    - introduction dans la membrane de ligands hydrophobes choisis parmi $C_1$-$C_{20}$-alkyle et dérivés de ceux-ci ou $C_6$-$C_{25}$-aryle et dérivés de ceux-ci ou $C_7$-$C_{25}$-arylalkyle et dérivés de ceux-ci ou -[$(CH_2)_m$-O-]$_n$-R, m étant 2 ou 3, n étant un nombre entier plus grand ou égal à 1 et R étant -H ou -$C_1$-$C_5$-alkyle.

2.  Procédé selon la revendication 1 dans lequel le milieu de saponification comporte au moins un additif influençant le gonflement.

3.  Procédé selon la revendication 1 ou 2 dans lequel la température du milieu de saponification pendant la saponification est de 10 °C au point d'ébullition dudit milieu de saponification.

4.  Procédé selon l'une ou plusieurs des revendications 1 à 3 dans lequel la saponification est effectuée dans une période de temps allant de 0,1 à 60 min.

5.  Procédé selon l'une ou plusieurs des revendications 1 à 4 dans lequel l'agent de réticulation est choisi dans le groupe comportant des composés diépoxydes, diisocyanates, épichlorhydrines, epibromhydrines, diméthylurées, diméthyléthylènurées, diméthylchlorosilanes, bis-(2-hydroxyéthylsufones), divinylsulfones, alkylène-dihalogènes, hydroxyalkylène-dihalogènes et glycidyl ethers.

6.  Procédé selon l'une ou plusieurs des revendications 1 à 5 dans lequel la réticulation est effectuée en milieu aqueux, dans un solvant organique ou dans un mélange d'eau et de solvant organique.

7.  Procédé selon l'une ou plusieurs des revendications 1 à 6 dans lequel la réticulation est effectuée en présence d'un catalyseur de réticulation.

8.  Procédé selon l'une ou plusieurs des revendications 1 à 7 dans lequel la réticulation est effectuée à une température allant d'environ 4 °C au point d'ébullition du milieu de réticulation.

9.  Procédé selon l'une ou plusieurs des revendications 1 à 8 dans lequel la réticulation est effectuée dans une période de temps allant de 10 min à 100 heures.

10. Procédé selon l'une ou plusieurs des revendications 1 à 9 dans lequel les ligands hydrophobes sont introduits pendant ou après la réticulation.

**11.** Procédé selon la revendication 10 dans lequel la membrane est séchée après introduction des ligands hydrophobes.

**12.** Procédé selon la revendication 11 dans lequel la membrane est séchée à partir d'un milieu comportant un composé stabilisant pour les pores.

**13.** Membrane d'hydrate de cellulose réticulé présentant une structure double poreuse, obtenue selon le procédé de la revendication 1, ladite membrane étant constituée

- de micropores ayant un diamètre dans la gamme allant de >100 nm à 20 $\mu$m, et
- d'ultra-pores ayant un diamètre de <100 nm qui ne sont pas accessibles au bleu de dextrane ayant un poids moléculaire moyen de 2000000,

la fraction volumétrique des ultra-pores par rapport au volume total de pores accessibles à l'eau étant de plus de 15% et

des ligands hydrophobes choisis parmi $C_1$-$C_{20}$-alkyle et dérivés de ceux-ci ou $C_6$-$C_{25}$-aryle et dérivés de ceux-ci ou $C_7$-$C_{25}$-arylalkyle et dérivés de ceux-ci ou $-[(CH_2)_m$-O-$]_n$-R, m étant 2 ou 3, n étant un nombre entier plus grand ou égal à 1 et R étant -H ou - $C_1$-$C_5$-alkyle, sont liés à la membrane.

**14.** Dispositif pour la chromatographie hydrophobe par interaction, comportant au moins une membrane d'hydrate de cellulose réticulé selon la revendication 13.

**15.** Utilisation de la membrane d'hydrate de cellulose réticulé selon la revendication 13 dans la chromatographie hydrophobe par interaction.

**16.** Utilisation de la membrane d'hydrate de cellulose réticulé selon la revendication 13 pour l'élimination de contaminants.

**17.** Utilisation de la membrane d'hydrate de cellulose réticulé selon la revendication 13 pour l'adsorption sélective suivie d'une désorption d'une ou plusieurs substances cibles.

Figur 1

a)                          b)                          c)

Träger    Adsorbend        Polymerbeschichtung         Ultraporen

Figur 2

Durchlaufende
Mikroporen

grobe Struktur aus relativ
dicken Fasern der
Cellulose bzw. deren
Agglomeraten, adsorptiv
unwirksam

feiner verteiltem, faser-
oder clusterartigen
Membranmaterial

An der adsorptiv
wirksamen
Polymerschicht
gebundenes Lysozym

Figur 3

| Durchlaufende | grobe Struktur aus relativ | feiner verteiltem, faser- |
| Mikroporen | dicken Fasern der | oder clusterartigen |
| | Cellulose bzw. deren | Membranmaterial mit |
| | Agglomeraten, mit | gebundenem Lysozym |
| | gebundenem Lysozym | |

Figur 4

| Durchlaufende Mikroporen | grobe Struktur aus relativ dicken Fasern der Cellulose bzw. deren Agglomeraten mit für Biomoleküle zugänglichen Ultraporen mit gebundenem Lysozym | feiner verteiltem, faser- oder clusterartigen Membranmaterial mit für Biomoleküle zugänglichen Ultraporen mit gebundenem Lysozym |

EP 2 274 080 B1

**Figur 5:**

Figur 6: Proteintrennung nach Beispiel 15 mit Butyl-Membran

Figur 7: Proteintrennung nach Beispiel 15 mit Phenyl-Membran

Figur 8: Proteintrennung nach Beispiel 15 mit Hexyl-Membran

Figur 9: Proteintrennung nach Beispiel 15 mit Octyl-Membran

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4323913 A1
- WO 03015902 A2
- US 6103121 A
- WO 2007017085 A2
- WO 2008095709 A1

**In der Beschreibung aufgeführte Nicht-Patentliteratur**

- **VON KUBOTA.** Control of phenyl-group site introduced on the graft chain for hydrophobic interaction chromatography. *Reactive & Functional Polymers,* 1996, vol. 29, 115-122
- **VON KUBOTA.** Preparation of a hydrophobic porous membrane containing phenyl groups and its protein adsorption performance. *Journal of Chromatography A,* 1995, vol. 718, 27-34
- **VON KAWAI.** Protein binding to polymer brush, based on ion-exchange, hydrophobic and affinity interactions. *Journal of Chromatography B,* 2003, vol. 790, 131-142
- **VON SAITO.** Protein adsorption capacity of a porous phenylalanine-containing membrane based on a polyethylene matrix. *Journal of Chromatography,* 1991, vol. 586, 27-33
- **VON GHOSH.** Purification of humanized monoclonal antibodies by membrane-based hybrid bioseparation technique. *Journal of Immunological Methods,* 2006, vol. 314, 1-8
- **E. MÜLLER.** *Chem. Eng. Technol.,* 2005, vol. 28 (11
- **GREG T. HERMANSON ; A. KRISHNA MALLIA ; PAUL K. SMITH.** Immobilized Affinity Ligand Techniques. Academic Press, INC, 1992